(19) Europäisches Patentamt
European Patent Office
Office européen des brevets

(11) Veröffentlichungsnummer: 0 201 735
B1

(12) EUROPÄISCHE PATENTSCHRIFT

(45) Veröffentlichungstag der Patentschrift: 12.09.90

(51) Int. Cl.[5]: C07D 209/16, C07D 209/14, C07D 401/12, A61K 31/40, C07D 409/12

(21) Anmeldenummer: 86105038.3

(22) Anmeldetag: 12.04.86

(54) N-Indolylethyl-sulfonsäureamide, Verfahren zu ihrer Herstellung und ihre Verwendung.

(30) Priorität: 24.04.85 DE 3514696

(43) Veröffentlichungstag der Anmeldung: 20.11.86 Patentblatt 86/47

(45) Bekanntmachung des Hinweises auf die Patenterteilung: 12.09.90 Patentblatt 90/37

(84) Benannte Vertragsstaaten: AT BE CH DE FR GB IT LI NL SE

(56) Entgegenhaltungen:
JOURNAL OF PHARMACEUTICAL SCIENCES, Band 60, Nr. 4, April 1971, Seiten 636-637; B.T. HO et al.: "Hydroxyindole-O-methyltransferase VI: Inhibitory activities of substituted benzoyltryptamines and benzenesulfonyltryptamines" Seiten 636-637
JOURNAL OF MEDICINAL CHEMISTRY, Band 14, Nr. 6, Juni 1971, Seiten 553-554; B.T. HO et al.: "Central nervous system depressive activity of some amides of tryptamine"
CHEMICAL ABSTRACTS, Band 94, Nr. 19, 11. Mai 1981, Seite 637, nr. 156681z, Columbus, Ohio, US; D.R. LAGIDZE et al.: "Synthesis of some new analogs of melatonin and beta-carboline from 4-phenylpentanoic acid"
CHEMICAL ABSTRACTS, Band 87, Nr. 19, 7. November 1977, Seite 255, Nr. 148178f, Columbus, Ohio,

(73) Patentinhaber: BAYER AG, D-5090 Leverkusen 1 Bayerwerk(DE)

(72) Erfinder: Böshagen, Horst, Dr., Wiesenstrasse 4, D-5657 Haan(DE)
Erfinder: Rosentreter, Ulrich, Dr., Kondorweg 22, D-5600 Wuppertal 1(DE)
Erfinder: Lieb, Folker, Dr., Alfred-Kubin-Strasse 1, D-5090 Leverkusen(DE)
Erfinder: Oediger, Hermann, Dr., Roggendorfstrasse 51, D-5000 Koeln 80(DE)
Erfinder: Seuter, Friedel, Dr., Moospfad 16, D-5600 Wuppertal 1(DE)
Erfinder: Perzborn, Elisabeth, Dr., Am Tescher Busch 13, D-5600 Wuppertal 11(DE)
Erfinder: Fiedler, Volker-Bernd, Dr., Lehner Mühle 46, D-5090 Leverkusen 3(DE)

(56) Entgegenhaltungen: (Fortsetzung)
US; M. WIECHMANN: "Scope and limitations of the analytical use of dansyl chloride. I. The reaction of aromatic sulfonyl chlorides with aliphatic tertiary amines: the microanalytical aspects of the Hinsberg test" 000
JOURNAL OF THE CHEMICAL SOCIETY, PERKIN TRANSACTIONS I, 1973, Seiten 1602-1606; A.S. BAILEY et al.: "Further examination of the reactions of simple indoles with arenesulphonyl azides"
CHEMICAL ABSTRACTS, Band 94, Nr. 19, 11. Mai 1981, Seite 637, nr. 156681z, Columbus, Ohio, US; D.R. LAGIDZE et al.: "Synthesis of some new analogs of melatonin and beta-carboline from 4-phenylpentanoic acid"
CHEMICAL ABSTRACTS, Band 87, Nr. 19, 7. November 1977, Seite 255, Nr. 148178f, Columbus, Ohio, US; M. WIECHMANN: "Scope and limitations of the analytical use of dansyl chloride. I. The reaction of aromatic sulfonyl chlorides with aliphatic tertiary amines: the microanalytical aspects of the Hinsberg test" & HOPPE-SEYLER cS Z. PHYSIOL. CHEM. 1977, 358(8), 967-80

Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 201 735 B1

ACTORUM AG

## Beschreibung

Die Erfindung betrifft neue N-Indolylethyl-sulfonsäureamide, Verfahren zu ihrer Herstellung und ihre Verwendung als Wirkstoffe in Arzneimitteln.

Es wurden neue N-Indolylethyl-sulfonsäureamide der Formel

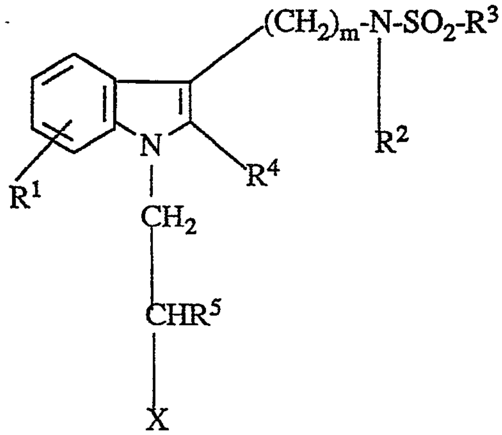

(I),

in der

$R^1$ Wasserstoff, Fluor, Chlor, Brom, Trifluormethyl, Carboxy, Niederalkoxy-($C_1$ bis $C_6$)carbonyl, die Gruppe

$$-S(O)_nR^6$$

in der

$R^6$ Niederalkyl ($C_1$ bis $C_6$) oder Phenyl und

n eine der Zahlen 0, 1 oder 2 bedeutet,

die Gruppe

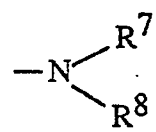

in der

$R^7$ und $R^8$ gleich oder verschieden sind und Wasserstoff, Niederalkyl ($C_1$ bis $C_6$), Phenyl, Benzyl oder Acetyl bedeuten, oder

die Gruppe

$$-O-R^9$$

in der

$R^9$ Wasserstoff, Niederalkyl ($C_1$ bis $C_6$), Phenyl, $C_7$ bis $C_{10}$-Aralkyl, $-SO_2$-Niederalkyl ($C_1$ bis $C_6$), $-SO_2$-Phenyl, $-SO_2$-Benzyl oder Trifluormethyl bedeutet, oder

gegebenenfalls durch Carboxy, Niederalkoxy ($C_1$ bis $C_6$)carbonyl, Fluor, Chlor, Brom und/oder Cyano substituiertes Niederalkyl ($C_1$ bis $C_6$), Niederalkenyl ($C_2$ bis $C_6$), Cyclopentyl oder Cyclohexyl bedeutet,

$R^2$ Wasserstoff bedeutet,

$R^3$ Niederalkyl ($C_1$ bis $C_6$), Niederalkenyl ($C_2$ bis $C_6$), Cyclopentyl, Cyclohexyl, gegebenenfalls bis zu vierfach durch Fluor, Chlor, Brom, Cyano, Hydroxy, Trifluormethyl, Trifluormethoxy, Niederalkyl ($C_1$ bis $C_6$), Niederalkenyl ($C_2$ bis $C_6$), die selbst wiederum durch Carboxy oder $C_1$ bis $C_4$-Alkoxycarbonyl substituiert sein können, Niederalkoxy ($C_1$ bis $C_6$), Carboxyl, $C_1$ bis $C_4$-Alkoxycarboxyl, Benzyloxy, Benzylthio, Phenyl, Phenoxy und/oder die Gruppe

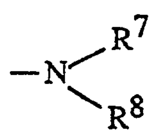

in der

$R^7$ und $R^8$ die obengenannte Bedeutung haben,

gegebenenfalls substituiertes Phenyl oder Naphthyl, oder gegebenenfalls bis zu vierfach durch Fluor, Chlor, Brom, Amino, Dimethylamino, Acetylamino, Trifluormethyl, Trifluormethoxy, $C_1$ bis $C_4$-Alkyl und/oder $C_1$ bis $C_4$-Alkoxy substituiertes Pyridyl, Thienyl, Furyl, Pyrimidyl, Piperazinyl, Piperidinyl, Morpholinyl, Thiomorpholinyl, Chinolyl, Isochinolyl, Imidazolyl, Triazolyl, Tetrazolyl, Thiadiazolyl, Pyrrolyl, Benzimidazolyl, Benzothiadiazolyl, Indolyl, Indolonyl, Thiazolyl, Isothiazolyl, Benzthiazolyl, Benzoisothiazolyl, Benzoisothiazolonyl bedeutet,
$R^4$ Wasserstoff, Niederalkyl ($C_1$ bis $C_6$), Niederhydroxyalkyl ($C_1$ bis $C_6$), Niederalkenyl ($C_2$ bis $C_6$), Niederalkyl ($C_1$ bis $C_6$)carbonyl, Cyano, gegebenenfalls bis zu dreifach durch Fluor, Chlor, Methyl, Methoxy und/oder Trifluormethyl substituiertes Phenyl, Pyridyl, Thienyl, Furyl, Pyrimidyl, Imidazolyl oder Pyrrolyl bedeutet,
$R^5$ Wasserstoff oder Niederalkyl($C_1$ bis $C_6$) bedeutet,
X Carboxy bedeutet und
m die Zahl 2 bedeutet,
und deren Salze gefunden.

Bevorzugte Bedeutung von $R^1$ ist
Wasserstoff, Fluor, Chlor, Brom, Trifluormethyl,
die Gruppe

$$-S(O)_n-R^6$$

in der
$R^6$ Methyl, Ethyl oder Phenyl und
n eine der Zahlen 0 oder 2 bedeutet,
Amino, Methylamino, Dimethylamino, Acetylamino,
die Gruppe

$$-O-R^9$$

in der
$R^9$ Wasserstoff, $C_1$ bis $C_4$-Alkyl, Phenyl oder Benzyl bedeutet, oder
gegebenenfalls durch Fluor, Chlor und/oder Cyano substituiertes $C_1$ bis $C_4$-Alkyl.

Im besonderen bevorzugt wird eine Substitution des Restes $R^1$ in 5-Stellung des Indolsystems.

Erfindungsgemäß hat der Rest $R^2$ die Bedeutung Wasserstoff.

Bevorzugte Bedeutung von $R^3$ ist
$C_1$ bis $C_4$-Alkyl, gegebenenfalls bis zu dreifach durch Fluor, Chlor, Brom, Cyano, Hydroxy, Trifluormethyl, $C_1$ bis $C_4$-Alkyl, Carboxyvinyl, $C_1$ bis $C_4$-Alkoxy, $C_1$- bis $C_4$-Meth- oder -Ethoxycarbonylalkyl, Amino, Dimethylamino, Acetylamino, Phenyl und/oder Phenoxy substituiertes Phenyl, Naphthyl, oder durch $C_1$ bis $C_4$-Alkyl, Fluor, Chlor, Amino, Dimethylamino substituiertes Pyridyl, Thienyl, Furyl, Pyrimidyl, Piperazinyl, Piperidinyl, Morpholinyl, Chinolyl, Benzthiadiazolyl, Benzisothiazolonyl oder Indolonyl.

Bevorzugte Bedeutung von $R^5$ ist
$C_1$ bis $C_4$-Alkyl, Wasserstoff.

Bevorzugte Bedeutung von $R^4$ ist
Wasserstoff, Methyl, Ethyl, Methylcarbonyl, Cyano, Hydroxymethyl, Hydroxyethyl oder Phenyl.

Erfindungsgemäß hat der Rest X die Bedeutung Carboxy.

Erfindungsgemäße Bedeutung von m ist 2.

Alkyl steht im allgemeinen erfindungsgemäß für einen geradkettigen oder verzweigten Kohlenwasserstoffrest mit 1 bis 8 Kohlenstoffatomen. Bevorzugt wird Niederalkyl mit 1 bis etwa 6 Kohlenstoffatomen. Insbesondere bevorzugt wird ein Alkylrest mit 1 bis 4 Kohlenstoffatomen. Beispielsweise seien die folgenden Alkylreste genannt:
Methyl, Ethyl, Propyl, Isopropyl, Butyl, Isobutyl, Pentyl, Isopentyl, Hexyl, Isohexyl, Heptyl, Isoheptyl, Octyl und Isooctyl.

Alkenyl steht im allgemeinen für einen geradkettigen oder verzweigten Kohlenwasserstoffrest mit 2 bis 8 Kohlenstoffatomen und einer oder mehreren, bevorzugt einer oder zwei, Doppelbindungen. Bevorzugt ist ein Niederalkenylrest mit 2 bis etwa 6 Kohlenstoffatomen und einer Doppelbindung. Insbesondere bevorzugt ist ein Alkenylrest mit 1 bis 4 Kohlenstoffatomen und einer Doppelbindung. Beispielsweise seien die folgenden Alkenylreste genannt: Vinyl, Allyl, Propenyl, Isopropenyl, Butenyl, Isobutenyl, Pentenyl, Isopentenyl, Hexenyl, Isohexenyl, Heptenyl, Isoheptenyl, Octenyl und Isooctenyl.

Alkoxy steht im allgemeinen erfindungsgemäß für eine über Sauerstoff gebundene, geradkettige oder verzweigte Kohlenwasserstoffkette mit 1 bis 8 Kohlenstoffatomen. Bevorzugt ist Niederalkoxy mit 1 bis 6 Kohlenstoffatomen. Insbesondere bevorzugt ist ein Alkoxyrest mit 1 bis 4 Kohlenstoffatomen. Beispielsweise seien die folgenden Alkoxyreste genannt: Methoxy, Ethoxy, Propoxy, Isopropoxy, Butoxy, Isobutoxy, Pentoxy, Isopentoxy, Hexoxy, Isohexoxy, Heptoxy, Isoheptoxy, Octoxy und Isooctoxy.

Cycloalkyl steht im allgemeinen erfindungsgemäß für einen cyclischen Kohlenwasserstoffrest mit 5 bis 8 Kohlenstoffatomen. Bevorzugt ist der Cyclopentan- und der Cyclohexanrest. Beispielsweise seien die folgenden Cycloalkylreste genannt: Cyclopentyl, Cyclohexyl, Cycloheptyl und Cyclooctyl.

Aryl steht im allgemeinen erfindungsgemäß für einen aromatischen Rest mit 6 bis 12 Kohlenstoffatomen. Bevorzugte Arylreste sind Phenyl, Naphthyl und Biphenyl.

Aralkyl steht im allgemeinen erfindungsgemäß für einen über eine Alkylenkette gebundenen Arylrest mit 7 bis 14 Kohlenstoffatomen. Bevorzugt werden Aralkylreste mit 1 bis 6 Kohlenstoffatomen im aliphatischen Teil und 6 bis 12 Kohlenstoffatomen im aromatischen Teil. Beispielsweise seien die folgenden Aralkylreste genannt: Benzyl, Naphthylmethyl, Phenethyl.

Alkoxycarbonyl kann beispielsweise durch die Formel

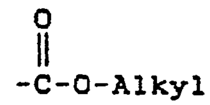

dargestellt werden. Alkyl steht hierbei für einen geradkettigen oder verzweigten Kohlenwasserstoffrest mit 1 bis 8 Kohlenstoffatomen. Bevorzugt wird Niederalkoxycarbonyl mit 1 bis 6 Kohlenstoffatomen im Alkylteil. Insbesondere bevorzugt wird ein Alkoxycarbonylrest mit 1 bis 4 Kohlenstoffatomen im Alkylteil. Beispielsweise seien die folgenden Niederalkoxycarbonylreste genannt: Methoxycarbonyl, Ethoxycarbonyl, Propoxycarbonyl, Isopropoxycarbonyl, Butoxycarbonyl, Isobutoxycarbonyl.

Halogen steht im allgemeinen erfindungsgemäß für Fluor, Chlor, Brom oder Iod, bevorzugt für Fluor, Chlor oder Brom, insbesondere bevorzugt für Fluor oder Chlor.

Die Reste $R^1$, $R^2$, $R^3$, $R^4$ und X können gegebenenfalls in anderen Resten substituiert sein. Bevorzugt sind Substitutionen bis zu 4 anderen Resten, besonders bevorzugt mit bis zu 3 anderen Resten und insbesondere bevorzugt mit bis zu 2 anderen Resten.

Die erfindungsgemäßen N-Indolylethyl-sulfonsäureamide können auch in Form ihrer Salze vorliegen. Im allgemeinen seien hier Salze mit organischen oder anorganischen Basen genannt.

Im Rahmen der vorliegenden Erfindung werden physiologisch unbedenkliche Salze bevorzugt. Physiologisch unbedenkliche Salze der N-Indolylethyl-sulfonsäureamide können Metall- oder Ammoniumsalze der erfindungsgemäßen Stoffe, welche eine freie Carboxylgruppe besitzen, sein. Besonders bevorzugt sind z.B. Natrium-, Kalium-, Magnesium- oder Calciumsalze, sowie Ammoniumsalze, die abgeleitet sind von Ammoniak oder organischen Aminen, wie beispielsweise Ethylamin, Di- bzw. Triethylamin, Di- bzw. Triethanolamin, Dicyclohexylamin, Dimethylaminoethanol, Arginin oder Ethylendiamin.

Beispielsweise seien die folgenden N-Indolylethyl-sulfonsäureamide genannt:

N-[2-[1-(2-Carboxyethyl)-1H-indol-3-yl]]ethyl-(4-methyl-phenyl)sulfonamid,
N-[2-[1-(2-Carboxyethyl)-5-methoxy-1H-indol-3-yl]]ethyl-phenylsulfonamid-natriumsalz,
N-[2-[1-(2-Carboxyethyl)-5-hydroxy-1H-indol-3-yl]]ethyl-phenylsulfonamid-natriumsalz,
N-[2-[5-Benzyloxy-1-(2-carboxyethyl)-1H-indol-3-yl]]ethyl-phenylsulfonamid,
N-[2-[1-(2-carboxyethyl)-1H-indol-3-yl]]-ethyl-(4-chlorphenyl)sulfonamid-Triethylaminsalz,
N-[2-[1-(2-carboxylethyl)-1H-indol-3-yl]]ethyl-(2,5-dichlorphenyl)sulfonamid,
N-[2-[1-(2-Carboxyethyl)-2-methyl-1H-indol-3-yl]]ethyl-phenylsulfonamid,
N-[2-[1-(2-Carboxyethyl)-1H-indol-3-yl]]ethyl-(2,4-dichlorphenyl)-sulfonamid,
N-[2-[1-(2-Carboxyethyl)-1H-indol-3-yl]]ethyl-2-thienylsulfonamid,
N-[2-[1-(2-Carboxyethyl)-1H-indol-3-yl]]ethyl-(4-phenoxyphenyl)sulfonamid,
N-[2-[1-(2-Carboxyethyl)-1H-indol-3-yl]]ethyl-3-pyridylsulfonamid.

Gegenstand der Erfindung ist auch ein Verfahren zur Herstellung von N-Indolylethyl-sulfonsäureamiden der Formel (I), bzw. deren Salze, das dadurch gekennzeichnet ist, daß man Indolylalkylamine der Formel

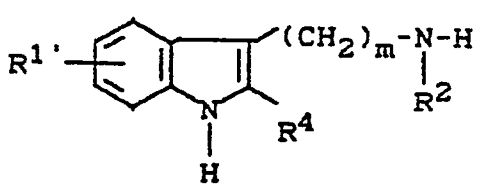

(II),

in der

$R^{1'}$ Wasserstoff, Fluor, Chlor, Brom, Trifluormethyl, Carboxy, Niederalkoxy($C_1$ bis $C_6$)carbonyl, die Gruppe

$$-S(O)_n-R^6$$

in der

$R^6$ Niederalkyl ($C_1$ bis $C_6$) oder Phenyl und
n eine der Zahlen 0, 1 oder 2 bedeutet,
die Gruppe

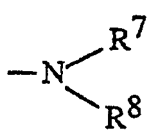

in der

$R^7$ und $R^8$ gleich oder verschieden sind und Wasserstoff, Niederalkyl ($C_1$ bis $C_6$), Phenyl, Benzyl oder Acetyl bedeuten, oder
die Gruppe

$-O-R^9$

in der $R^9$ Niederalkyl, ($C_1$ bis $C_6$) Phenyl, $C_7$ bis $C_{10}$-Aralkyl, $-SO_2$-Niederalkyl ($C_1$ bis $C_6$), $-SO_2$-Phenyl, $-SO_2$-Benzyl oder Trifluormethyl bedeutet, oder gegebenenfalls durch Carboxy, Niederalkoxy ($C_1$ bis $C_6$)carbonyl, Fluor, Chlor, Brom und/oder Cyano substituiertes Niederalkyl ($C_1$ bis $C_6$), Niederalkenyl ($C_2$ bis $C_6$), Cyclopentyl oder Cyclohexyl bedeutet, und $R^2$, $R^4$ und m die oben angegebene Bedeutung haben, mit Sulfonsäurehalogeniden der Formel

$$R^3-SO_2-Y \qquad (III),$$

in der $R^3$ die oben angegebene Bedeutung hat und Y für Halogen steht, in Gegenwart eines inerten Lösungsmittels, gegebenenfalls in Gegenwart einer Base, umsetzt, dann mit einer olefinischen Verbindung der Formel

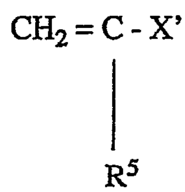

(IV),

in der X' Niederalkoxy($C_1$ bis $C_6$)carbonyl oder Cyano bedeutet, und $R^5$ die oben angegebene Bedeutung hat, in Gegenwart eines inerten Lösungsmittels, gegebenenfalls in Gegenwart einer Base zu den Zwischenverbindungen der Formel Ia

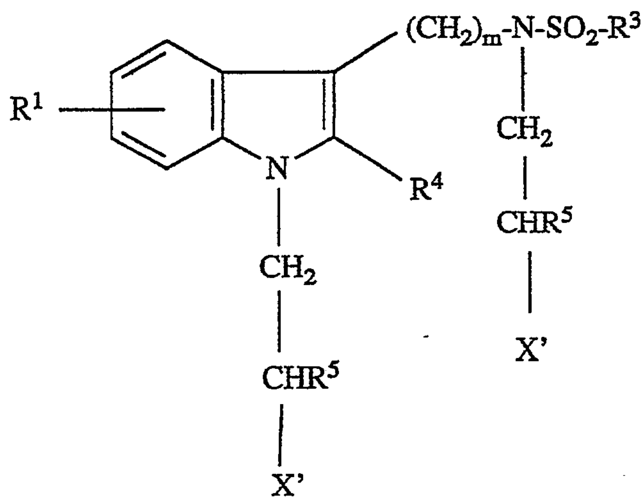

(Ia)

alkyliert, dann für den Fall der Herstellung von in 4-, 5-, 6- oder 7-Stellung durch Hydroxy substituierten Verbindungen die entsprechenden Benzyloxyverbindungen in Gegenwart eines Katalysators in einem inerten Lösungsmittel, gegebenenfalls in Gegenwart einer Säure hydriert, dann für den Fall der Herstellung von N-Indolylethylcarboxyl-Verbindungen die entsprechenden Nitrile oder Alkoxycarbonyle verseift, und dann für den Fall der Herstellung von Salzen mit einer entsprechenden Base umsetzt.

Das erfindungsgemäße Verfahren kann beispielsweise durch das folgende Formelschema erläutert werden:

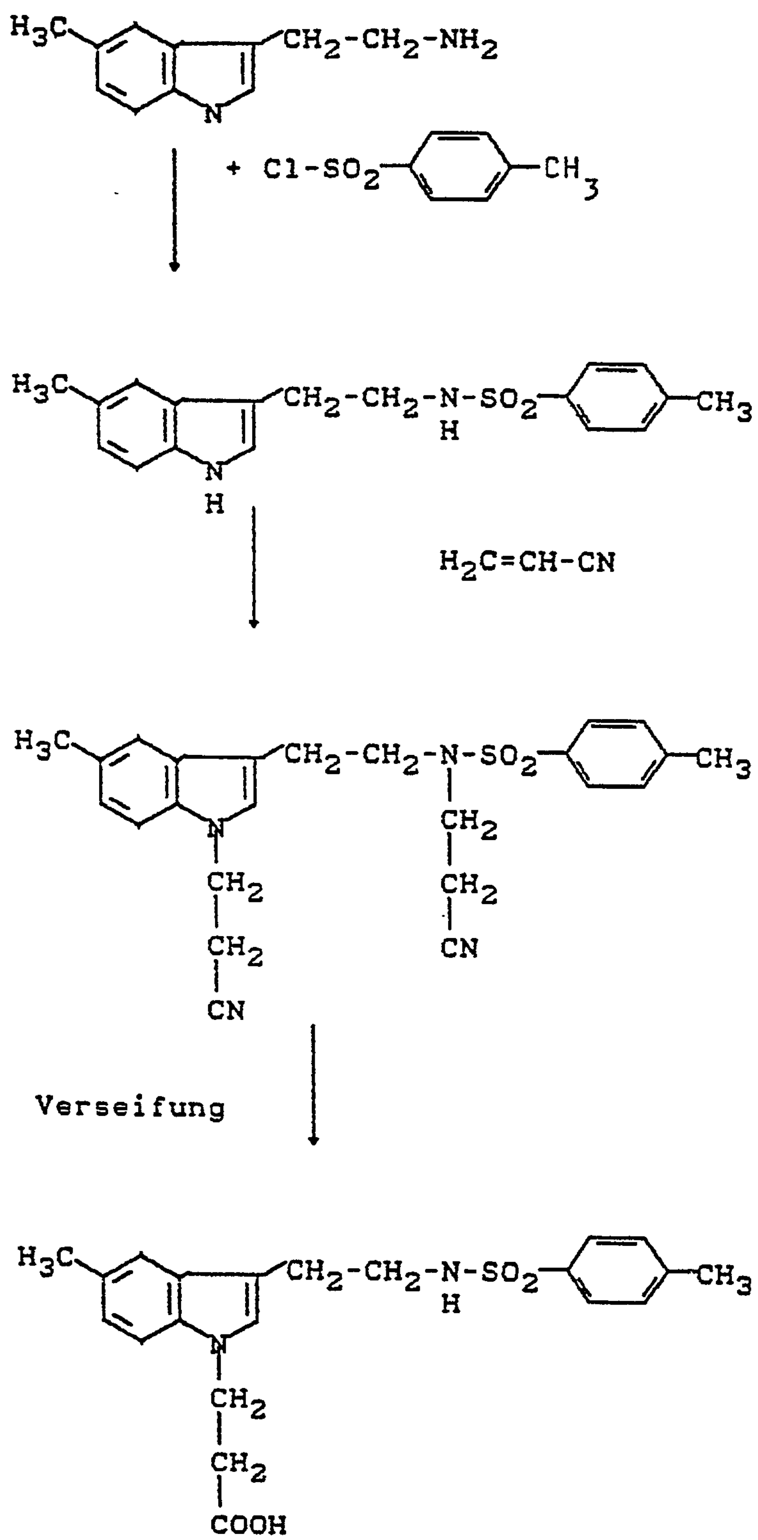


Bei der Durchführung des erfindungsgemäßen Verfahrens entstehen zum Beispiel Zwischenprodukte der Formel (Ia), die isoliert werden können. So ist es möglich, das erfindungsgemäße Verfahren in mehreren Verfahrensstufen auszuführen. Es kann aber auch möglich sein, verschiedene Verfahrensschritte zu kombinieren und als Eintopfverfahren auszuführen.

Die Indolylalkylamine (Formel II), die Sulfonsäurehalogenide (Formel III) und die olefinischen Verbindungen (Formel IV) sind an sich bekannt oder können nach an sich bekannten Methoden hergestellt werden (Houben-Weyl, Methoden der organischen Chemie, Band 9, 407 ff und 547 ff (1959); The Chemistry of Indoles, Academic Press (1970); W.J. Houlihan, Indoles Part Two, John Wiley and Sons (1972)).

Lösungsmittel für das erfindungsgemäße Verfahren können inerte organische Lösungsmittel sein, die sich unter den Reaktionsbedingungen nicht verändern. Hierzu gehören bevorzugt Alkohole, wie beispielsweise Methanol, Ethanol, n-Propanol, iso-Propanol, Ether wie beispielsweise Diethylether, Dioxan, Tetrahydrofuran, Halogenkohlenwasserstoffe wie Di-, Tri- oder Tetrachlormethan, Dichlorethylen, Trichlorethylen, Essigester, Toluol, Acetonitril, Nitromethan, Dimethylformamid, Hexamethylphosphorsäuretriamid, Pyridin und Aceton. Selbstverständlich ist es möglich, Gemische der Lösungsmittel einzusetzen.

Als Basen für das erfindungsgemäße Verfahren können übliche basische Verbindungen für basische Reaktionen sein. Hierzu gehören vorzugsweise Alkali- und Erdalkalihydroxide oder -carbonate wie beispielsweise Lithium-, Natrium-, Kalium-, Calcium- oder Bariumhydroxid, Natrium- oder Kaliumcarbonat, Alkalialkoholate wie beispielsweise Natriummethylat und -ethylat oder Kaliummethylat und -ethylat, oder organische Basen wie beispielsweise Triethylamin, Pyridin oder 1-Methylpiperidin, Benzyltrimethylammoniumhydroxid oder Tetrabutylammoniumhydroxid.

Das erfindungsgemäße Verfahren wird im allgemeinen im Temperaturbereich von -20 bis +100°C, bevorzugt von 0°C bis 80°C, durchgeführt.

Das erfindungsgemäße Verfahren wird im allgemeinen bei Normaldruck durchgeführt. Es ist aber auch möglich, das Verfahren bei Unter- oder Überdruck durchzuführen (beispielsweise im Druckbereich von 1 bis 10 bar.

Im allgemeinen setzt man 1 bis 5 Mol, bevorzugt 1 bis 2 Mol, besonders bevorzugt 1 Mol Sulfonsäurehalogenid bezogen auf 1 Mol des Indolylalkylamins ein. Die olefinische Verbindung wird im allgemeinen in einer Menge von 1 bis 10 Mol, bevorzugt 1 bis 5 Mol, besonders bevorzugt 3 Mol, bezogen auf 1 Mol des Indolylalkylamins eingesetzt. Bei der Herstellung von N-Indolylethyl-sulfonsäureamiden die in 4-, 5-, 6- oder 7-Stellung durch Hydroxy substituiert sind, hydriert man die entsprechende Benzyloxyverbindung in Gegenwart eines Katalysators und eines inerten organischen Lösungsmittels, gegebenenfalls in Gegenwart einer Säure.

Als Lösungsmittel seien inerte organische Lösungsmittel genannt, die sich unter den Hydrierbedingungen nicht verändern. Hierzu gehören bevorzugt Alkohole wie Methanol, Ethanol, Propanol oder iso-Propanol, Ether wie Diethylether, Dioxan oder Tetrahydrofuran, Chlorkohlenwasserstoffe wie Di-, Tri- oder Tetrachlormethan, 1,2-Dichlorethan, Toluol oder Essigester.

Die Hydrierung wird bevorzugt in Gegenwart von Edelmetallkatalysatoren durchgeführt. Insbesondere bevorzugt sind Platin, Palladium oder Palladium/Tierkohlekatalysatoren. Der Katalysator wird im allgemeinen in einer Menge von 1 bis 100 Mol %, bevorzugt 5 bis 10 Mol %, bezogen auf die Benzyloxyindolylverbindung eingesetzt.

Als Säuren können erfindungsgemäß starke Mineralsäuren, aber auch organische Säuren eingesetzt werden. Bevorzugt sind Halogenwasserstoffsäuren wie Chlor- bzw. Bromwasserstoffsäure, Schwefelsäure, Phosphorsäure, Perchlorsäure, Essigsäure oder Trifluoressigsäure. Im allgemeinen setzt man 1 bis 100 Gew.-Teile, bevorzugt 1 bis 50 Gew.-Teile Säure, bezogen auf die Benzyloxyverbindungen, ein.

Die Hydrierung wird im allgemeinen im Temperaturbereich von -20 bis +100°C, bevorzugt im Bereich von 0 bis 50°C, durchgeführt.

Im allgemeinen wird die Hydrierung bei Normaldruck durchgeführt. Sie kann aber auch bei Über- oder Unterdruck durchgeführt werden (beispielsweise im Druckbereich von 0,5 bis 50 bar).

Für den Fall der Herstellung von N-Indolyl-ethyl-carboxy-verbindungen verseift man entsprechende Nitrile oder Alkoxycarbonyl-Verbindungen. Die Verseifung wird im allgemeinen in Gegenwart von Basen, bevorzugt Alkali- oder Erdalkali-hydroxide oder -alkoholate durchgeführt. Vorzugsweise werden Basen wie Alkali- oder Erdalkalihydroxide oder Alkalialkoholate, bevorzugt Lithium-, Natrium-, Kalium-, Calcium- oder Bariumhydroxid oder Natrium- bzw. Kaliummethylat oder -ethylat verwendet.

Im allgemeinen setzt man 1 bis 100 Mol, bevorzugt 2 bis 50 Mol, der Base bezogen auf 1 Mol des Nitrils oder der Alkoxycarbonylverbindung ein.

Als Indolylalkylamine für das erfindungsgemäße Verfahren seien beispielsweise genannt:
2-(5-Methyl-1H-indol-3-yl)ethylamin,
2-(2-Methyl-1H-indol-3-yl)ethylamin,
2-(5-Methoxy-1H-indol-3-yl)ethylamin,
2-(5-Benzyloxy-1H-indol-3-yl)ethylamin,
Tryptamin,
2-(2-Isopropyl-1H-indol-3-yl)ethylamin,
2-(2-tert.-Butyl-H-indol-3-yl)ethylamin.

Als Sulfonsäurehalogenide für das erfindungsgemäße Verfahren seien beispielsweise genannt: 4-Toluolsulfonylchlorid, 4-Chlorphenylsulfonylchlorid, 2,5-Dichlorphenylsulfonylchlorid, 3-Trifluormethylphenylsulphonylchlorid, 2,4-Dichlorphenylsulfonylchlorid, 4-Methoxyphenylsulfonylchlorid, 1-Napthylsulfonylchlorid, 2,4,6-Trimethylphenylsulfonylchlorid, Chinolyl-8-sulfonylchlorid, Thienyl-2-sulfonylchlorid, 2,6-Dichlorphenylsulfonylchlorid, 2,3,4-Trichlorphenylsulfonylchlorid.

Als olefinische Verbindungen für das erfindungsgemäße Verfahren seien beispielsweise genannt: Acrylnitril, Acrylsäuremethylester, Acrylsäureethylester, Methacrylnitril.

Das erfindungsgemäße Verfahren kann beispielsweise wie folgt durchgeführt werden:
Das Indolylalkylamin wird gelöst und tropfenweise mit dem Sulfonsäurehalogenid versetzt. Das bei der Reaktion entstehende Zwischenprodukt kann isoliert werden.

Die Umsetzung des Zwischenprodukts mit der olefinischen Verbindung erfolgt unter Rühren bei Raumtemperatur. Die Aufarbeitung erfolgt in an sich bekannter Weise.

Nach dem erfindungsgemäßen Verfahren erhält man N-Indolylethyl-sulfonsäureamide mit großer Reinheit und mit hohen Ausbeuten.

Die neuen N-Indolylethyl-sulfonsäureamide bzw. deren Salze können als Wirkstoffe in Arzneimitteln eingesetzt werden. Die Wirkstoffe weisen eine thrombozytenaggregationshemmende und Thromboxanantagonistische Wirkung auf. Sie können bevorzugt zur Behandlung von Thrombosen, Thromboembolien, Ischämien, als Antiasthmatika und als Antiallergika eingesetzt werden. Die neuen Wirkstoffe können in an sich bekannter Weise unter Verwendung inerter nichttoxischer, pharmazeutisch geeigneter Trägerstoffe oder Lösungsmittel in die üblichen Formulierungen überführt werden, wie Tabletten, Kapseln, Dragees, Pillen, Granulate, Aerosole, Sirupe, Emulsionen, Suspensionen und Lösungen. Hierbei soll die therapeutisch wirksame Verbindung jeweils in einer Konzentration von etwa 0,5 bis 90 Gew.-%, bevorzugt von 10 bis 70 Gew.-%, in der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nichttoxische organische Lösungsmittel wie Paraffine (z.B. Erdölfraktionen), pflanzliche Öle (z.B. Erdnuß-/Sesamöl), Alkohole (z.B. Ethylalkohol, Glycerin), Glykole (z.B. Propylenglykol, Polyethylenglykol) feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäureester, Polyoxyethylen-Fettalkohol -Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation kann in üblicher Weise erfolgen, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, beiintravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen. Bei oraler Applikation beträgt die Dosierung im allgemeinen etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehreren Einzelgaben über den Tag zu verteilen.

Die erfindungsgemäßen N-Indolylethyl-sulfonsäureamide können sowohl in der Humanmedizin als auch in der Veterinärmedizin angewendet werden.

Herstellungsbeispiele

Beispiel 1

N-[2-[1-(2-Carboxyethyl)-1H-indol-3-yl]]ethyl-(4-methylphenyl)sulfonamid

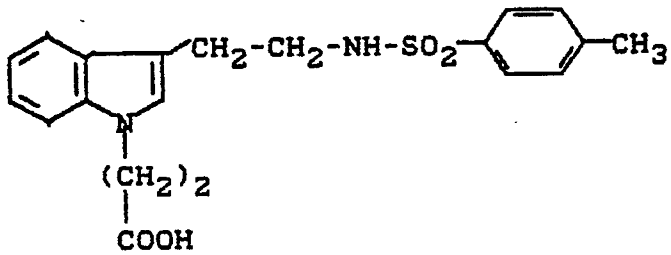

a) N-[2-(1H-Indol-3-yl)]ethyl-(4-methylphenyl)sulfonamid

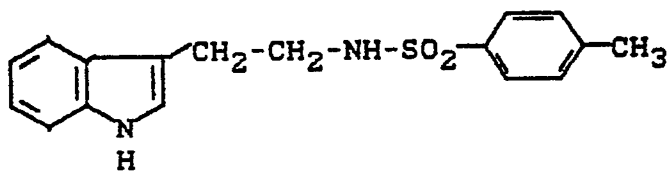

17.5 g (110 mmol) Tryptamin und 18.0 g (220 mmol) Natriumacetat werden in 250 ml Ethanol gelöst. Bei 0°C werden 20.97 g (110 mmol) p-Toluolsulfonsäurechlorid in 100 ml Ethanol gelöst zugetropft. Man rührt erst 1 h bei Raumtemperatur, dann 1 h bei Rückfluß. Anschließend gibt man so viel Wasser zu, bis eine klare Lösung entsteht. Ein Teil des Ethanols wird abdestilliert. Das Produkt kristallisiert aus, wird abgesaugt und aus Isopropanol umkristallisiert. Ausbeute: 20.7 g (60 % der Theorie)
Schmelzpunkt: 118°C
Rf-Wert: 0.6 in Toluol : Ethanol = 3:1

b) N-[2-Cyanoethyl]-N-[2-[1-(2-cyanoethyl)-1H-indol-3-yl]ethyl-(4-methylphenyl)sulfonamid

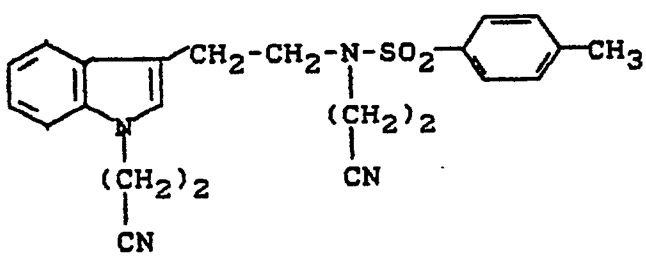

10 g 1a) werden in 150 ml Dioxan gelöst. Dann werden 10 ml Acrylnitril zugegeben. Zu dieser Lösung gibt man 1 ml Benzyltrimethylammoniumhydroxidlösung (40 %ig) gelöst in 4 ml Methanol. Es wird 5 h bei Raumtemperatur gerührt und dann in Wasser gegeben. Es wird dreimal mit Essigester extrahiert. Man wäscht die organische Phase mit Wasser, trocknet über $MgSO_4$, saugt ab und engt am Rotationsverdampfer ein. Mit wenig Ether kristallisiert das Produkt aus. Es wird aus Ethanol/Acetonitril (1:1) umkristallisiert. Ausbeute: 10.1 g (75.5 % der Theorie)
Schmelzpunkt: 88°C
Rf-Wert: 0.5 in Toluol : Ethanol = 3:1

c) N-[2-[1-(2-Carboxyethyl)-1H-indol-3-yl]]ethyl-(4-methylphenyl)sulfonamid

10 g 1b) werden in 300 ml 10 %iger Kalilauge 3 h bei Rückfluß gerührt. Dann stellt man mit 6 molarer HCl sauer und extrahiert dreimal mit Chloroform. Die organische Phase wird zweimal mit Wasser gewaschen, über $MgSO_4$ getrocknet und am Rotationsverdampfer eingeengt. Mit wenig Essigester kristallisiert das Produkt aus und wird abgesaugt.
Ausbeute: 4.7 g (51.1 % der Theorie)
Schmelzpunkt: 126°C

Beispiel 2

N-[2-[1-(2-Carboxyethyl)-5-methoxy-1H-indol-3-yl]]ethylphenylsulfonamid-Natriumsalz

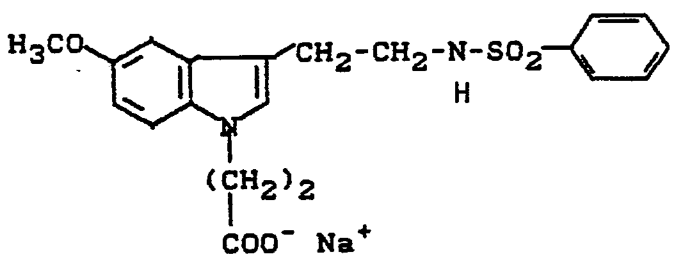

9.4 g N-[2-[1-(2-Carboxyethyl)-5-methoxy-1H-indol-3-yl]]-ethyl-phenylsulfonamid (hergestellt analog Beispiel 1) werden in 200 ml Methanol gelöst und mit 1.26 g Natriummethylat versetzt. Anschließend wird Methanol am Rotationsverdampfer abgezogen und der feste Rückstand mit Ether verrührt.
Ausbeute: 7.85 g (77 % der Theorie)
Schmelzpunkt: >225°C
Rf-Wert: 0.51 in $CH_2Cl$: Methanol = 9:1

Beispiel 3

N-[2-[1-(2-Carboxyethyl)-5-hydroxy-1H-indol-3-yl]]-ethyl-phenylsulfonamid-Natriumsalz

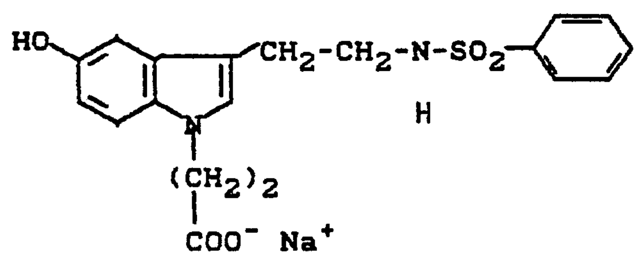

3 g N-[2-[5-Benzyloxy-1-(2-carboxyethyl)-1H-indol-3-yl]]-ethyl-phenylsulfonamid (hergestellt analog Beispiel 1) werden in einem Gemisch von 50 ml Ethanol und 15 ml Essigester gelöst und mit 200 mg 10 % Palladium auf Aktivkohle versetzt. Es wird so lange bei Normaldruck hydriert, bis die Wasserstoffaufnahme beendet ist. Dann wird der Katalysator abfiltriert, das Filtrat eingedampft und der Rückstand in Methylenchlorid gelöst. Die Methylenchloridlösung wird zweimal mit gesättigter Bicarbonatlösung extrahiert, die vereinigten Bicarbonatphasen mit 1 molarer Schwefelsäure sauer gestellt und mit Essigester extrahiert. Die Essigesterphase wird über $MgSO_4$ getrocknet und eingedampft. Man erhält so 1.5 g Öl, das analog Beispiel 2 in das Natriumsalz der freien Säure umgewandelt wird.
Ausbeute: 1.76 g (62.4 % der Theorie).
Schmelzpunkt: 100°C (Zers.).

Beispiele 4 bis 19

Nach der Arbeitsvorschrift von Beispiel 1 wurden die in der folgenden Tabelle aufgeführten Verbindungen hergestellt:

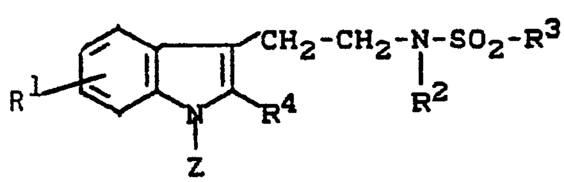

| Bsp. Nr. | $R^1$ | $R^2$ | $R^3$ | $R^4$ | Z | Schmelzpunkt (°C) | Rf-Wert in |
|---|---|---|---|---|---|---|---|
| 4a | H | $CH_2CH_2CN$ | $4\text{-}Cl\text{-}C_6H_4$ | H | $CH_2CH_2CN$ | 103 | 0,41 Toluol:Ethanol = 3:1 |
| b | H | H | $4\text{-}Cl\text{-}C_6H_4$ | H | $CH_2CH_2COOH \cdot NEt_3$ | 97 | 0,34 Toluol:Ethanol = 3:1 |
| 5a | H | $CH_2CH_2CN$ | $C_6H_5$ | H | $CH_2CH_2CN$ | 122 | 0,47 Toluol:Ethanol = 3:1 |
| b | H | H | $C_6H_5$ | H | $CH_2CH_2COOH \cdot NEt_3$ | 108 | 0,34 $CHCl_3$:Methanol = 10:1 |
| 6a | H | H | $2,5\text{-}Di\text{-}Cl\text{-}C_6H_3$ | H | H | 152 | 0,53 Toluol:Ethanol = 3:1 |
| b | H | $CH_2CH_2CN$ | " | H | $CH_2CH_2CN$ | Öl | 0,47 Toluol:Ethanol = 3:1 |
| c | H | H | " | H | $CH_2CH_2COOH$ | 132 | 0,42 Toluol:Ethanol = 3:1 |
| 7a | H | H | $C_6H_5$ | $CH_3$ | H | 136 | 0,5 Toluol:Ethanol = 3:1 |
| b | H | $CH_2CH_2CN$ | $C_6H_5$ | $CH_3$ | $CH_2CH_2CN$ | 145 | 0,49 Toluol:Ethanol = 3:1 |
| c | H | H | $C_6H_5$ | $CH_3$ | $CH_2CH_2COOH \cdot NEt_3$ | 127 | 0,22 $CHCl_3$:Methanol = 10:1 |
| 8a | H | H | $3\text{-}F_3C\text{-}C_6H_4$ | H | H | 104 | 0,52 Toluol:Ethanol = 3:1 |
| b | H | $CH_2CH_2CN$ | " | H | $CH_2CH_2CN$ | 133 | 0,51 Toluol:Ethanol = 3:1 |
| c | H | H | " | H | $CH_2CH_2COOH$ | 138 | 0,39 Toluol:Ethanol = 3:1 |
| 9a | H | H | $2,4,6\text{-}Tri\text{-}H_3C\text{-}C_6H_2$ | H | H | 139 | 0,55 Toluol:Ethanol = 3:1 |
| b | H | $CH_2CH_2CN$ | " | H | $CH_2CH_2CN$ | Öl | 0,51 Toluol:Ethanol = 3:1 |
| c | H | H | " | H | $CH_2CH_2COOH$ | 139 | 0,49 Toluol:Ethanol = 3:1 |
| 10a | H | H | 1-Piperidyl | H | H | 98 | 0,46 Toluol:Ethanol = 3:1 |
| b | H | $CH_2CH_2CN$ | " | H | $CH_2CH_2CN$ | Öl | 0,42 Toluol:Ethanol = 3:1 |
| c | H | H | " | H | $CH_2CH_2COOH$ | 55 | 0,5 Toluol:Ethanol = 3:1 |
| 11a | H | H | $2,4\text{-}Di\text{-}Cl\text{-}C_6H_3$ | H | H | 131 | 0,48 Toluol:Ethanol = 3:1 |
| b | H | $CH_2CH_2CN$ | " | H | $CH_2CH_2CN$ | Öl | 0,6 Toluol:Ethanol = 3:1 |
| c | H | H | " | H | $CH_2CH_2COOH$ | 89-95 | 0,32 Toluol:Ethanol = 3:1 |
| 12a | H | H | $4\text{-}H_3CO\text{-}C_6H_4$ | H | H | 115 | 0,45 Toluol:Ethanol = 3:1 |
| b | H | $CH_2CH_2CN$ | " | H | $CH_2CH_2CN$ | Öl | 0,56 Toluol:Ethanol = 3:1 |
| c | H | H | " | H | $CH_2CH_2COOH$ | 101 | 0,43 Toluol:Ethanol = 3:1 |
| 13a | H | H | $2,6\text{-}Di\text{-}Cl\text{-}C_6H_3$ | H | H | 142 | 0,64 Toluol:Ethanol = 3:1 |
| b | H | $CH_2CH_2CN$ | " | H | $CH_2CH_2CN$ | Öl | 0,46 Toluol:Ethanol = 3:1 |
| c | H | H | " | H | $CH_2CH_2COOH$ | 156 | 0,56 Toluol:Ethanol = 3:1 |

| Bsp. Nr. | $R^1$ | $R^2$ | $R^3$ | $R^4$ | Z | Schmelzpunkt (°C) | Rf-Wert in |
|---|---|---|---|---|---|---|---|
| 14a | H | H | 2-Naphthyl | H | H | 140 | 0,54 Toluol:Ethanol = 3:1 |
| b | H | $CH_2CH_2CN$ | " | H | $CH_2CH_2CN$ | 134 | 0,7 Toluol:Ethanol = 3:1 |
| c | H | H | " | H | $CH_2CH_2COOH$ | 137 | 0,55 Toluol:Ethanol = 3:1 |
| 15a | H | H | 2-Thienyl | H | H | 104 | 0,3 Toluol:Ethanol = 3:1 |
| b | H | $CH_2CH_2CN$ | " | H | $CH_2CH_2CN$ | 113 | 0,33 Toluol:Ethanol = 3:1 |
| c | H | H | " | H | $CH_2CH_2COOH$ | 115 | 0,31 Toluol:Ethanol = 3:1 |
| 16a | H | H | 2,3,4-Tri-Cl-$C_6H_2$ | H | H | 174 | 0,64 Toluol:Ethanol = 3:1 |
| b | H | $CH_2CH_2CN$ | " | H | $CH_2CH_2CN$ | Öl | 0,61 Toluol:Ethanol = 3:1 |
| c | H | H | " | H | $CH_2CH_2COOH$ | 135 | 0,49 Toluol:Ethanol = 3:1 |
| 17a | 5-$OCH_3$ | H | $C_6H_5$ | H | H | Öl | 0,44 Toluol:Ethanol = 9:1 |
| b | 5-$OCH_3$ | $CH_2CH_2CN$ | $C_6H_5$ | H | $CH_2CH_2CN$ | Öl | 0,5 Toluol:Ethanol = 9:1 |
| c | 5-$OCH_3$ | H | $C_6H_5$ | H | $CH_2CH_2COOH$ | $>225$ | 0,51 $CH_2O_2$:Methanol = 9:1 |
| 18a | 5-$OCH_2Ph$ | H | $C_6H_5$ | H | H | Öl | 0,31 Toluol:Ethanol = 9:1 |
| b | 5-$OCH_2Ph$ | $CH_2CH_2CN$ | $C_6H_5$ | H | $CH_2CH_2CN$ | Öl | 0,35 Toluol:Ethanol = 9:1 |
| c | 5-$OCH_2Ph$ | H | $C_6H_5$ | H | $CH_2CH_2COOH$ | 60-62 | 0,33 Toluol:Ethanol = 9:1 |
| 19a | 5-$CH_3$ | H | $C_6H_5$ | H | H | Öl | 0,81 $CH_2Cl_2$:Methanol = 9:1 |
| b | 5-$CH_3$ | $CH_2CH_2CN$ | $C_6H_5$ | H | $CH_2CH_2CN$ | Öl | 0,63 Toluol:Ethanol = 3:1 |
| c | 5-$CH_3$ | H | $C_6H_5$ | H | $CH_2CH_2COONa$ | 205-215 | 0,25 $CH_2Cl_2$:Methanol = 9,5:5 |
| 20a | H | H | 4-$H_5C_6$-O-$C_6H_4$ | H | H | 147 | 0,5 Toluol:Ethanol = 3:1 |
| b | H | $CH_2CH_2CN$ | 4-$H_5C_6$-O-$C_6H_4$ | H | $CH_2CH_2CN$ | Öl | 0,67 Toluol:Ethanol = 3:1 |
| c | H | H | 4-$H_5C_6$-O-$C_6H_4$ | H | $CH_2CH_2COOH$ | 110 | 0,26 Toluol:Ethanol = 3:1 |
| 21a | H | H | 3-Pyridyl | H | H | 110 | 0,92 $CHCl_3$:Methanol = 10:1 |

| Bsp. Nr. | $R^1$ | $R^2$ | $R^3$ | $R^4$ | Z | Schmelzpunkt (°C) | Rf-Wert | in |
|---|---|---|---|---|---|---|---|---|
| 21b | H | $CH_2CH_2CN$ | 3-Pyridyl | H | $CH_2CH_2CN$ | 110 | 0,56 | $CHCl_3$:Methanol = 10:1 |
| 21c | H | H | 3-Pyridyl | H | $CH_2CH_2COOH$ | 112 | 0,29 | $CHCl_3$:Methanol = 10:1 |
| 22a | H | H | 2-HO-3,5-Di-$CH_3$-$C_6H_2$ | H | H | 86-91 | 0,34 | Toluol:Ethanol = 3:1 |
| 22b | H | $CH_2CH_2CN$ | 2-HO-3,5-Di-$CH_3$-$C_6H_5$ | H | $CH_2CH_2CN$ | Öl | 0,52 | Toluol:Ethanol = 3:1 |
| 22c | H | H | 2-HO-3,5-Di-$CH_3$-$C_6H_5$ | H | $CH_2CH_2COOH \cdot NEt_3$ | 150-154 | 0,41 | Toluol:Ethanol = 3:1 |
| 23a | H | H | 8-Chinolyl | H | H | 140 | 0,38 | $CHCl_3$:Methanol = 10:1 |
| 23b | H | $CH_2CH_2CN$ | 8-Chinolyl | H | $CH_2CH_2CN$ | Öl | 0,74 | $CHCl_3$:Methanol = 10:1 |
| 23c | H | H | 8-Chinolyl | H | $CH_2CH_2COOH$ | 130 | 0,62 | $CHCl_3$:Methanol = 10:1 |
| 24a | H | H | $4(H_3C)_3C$-$C_6H_4$ | H | H | 151 | 0,53 | Toluol:Ethanol = 3:1 |
| 24b | H | $CH_2CH_2CN$ | $4(H_3C)_3C$-$C_6H_4$ | H | $CH_2CH_2CN$ | Öl | 0,68 | Toluol:Ethanol = 3:1 |
| 24c | H | H | $4(H_3C)_3C$-$C_6H_4$ | H | $CH_2CH_2COOH$ | 132 | 0,41 | Toluol:Ethanol = 3:1 |
| 25a | H | H | 4-$C_6H_5$-$C_6H_4$ | H | H | 184 | 0,53 | Toluol:Ethanol = 3:1 |
| 25b | H | $CH_2CH_2CN$ | 4-$C_6H_5$-$C_6H_4$ | H | $CH_2CH_2CN$ | 122 | 0,31 | Toluol:Essigester = 3:1 |
| 25c | H | H | 4-$C_6H_5$-$C_6H_4$ | H | $CH_2CH_2COOH$ | 164 | 0,44 | Toluol:Ethanol = 3:1 |
| 26a | H | H | 4HOOC-CH=CH-$C_6H_4$ | H | H | 244 | 0,25 | Toluol:Ethanol = 3:1 |
| 26b | H | $CH_2CH_2CN$ | 4HOOC-CH=CH-$C_6H_4$ | H | $CH_2CH_2CN$ | | | |
| 26c | H | H | 4HOOC-CH=CH-$C_6H_4$ | H | $CH_2CH_2COOH$ | | | |
| 27a | H | H | $CH_3$ | H | H | 138 | 0,78 | Toluol:Ethanol = 3:1 |
| 27b | H | $CH_2CH_2CN$ | $CH_3$ | H | $CH_2CH_2CN$ | 109 | 0,29 | Toluol:Essigester = 3:1 |
| 27c | H | H | $CH_3$ | H | $CH_2CH_2COOH$ | 128 | 0,25 | Toluol:Ethanol = 3:1 |
| 28a | H | H | $CH_2CH_3$ | H | H | 74 | 0,58 | Toluol:Ethanol = 3:1 |
| 28b | H | $CH_2CH_2CN$ | $CH_2CH_3$ | H | $CH_2CH_2CN$ | Öl | 0,37 | Toluol:Essigester = 3:1 |
| 28c | H | H | $CH_2CH_3$ | H | $CH_2CH_2COOH$ | | | |
| 29a | H | $CH_3$ | $C_6H_5$ | H | H | 110 | 0,44 | Toluol:Ethanol = 10:1 |
| 29b | H | $CH_3$ | $C_6H_5$ | H | $CH_2CH_2CN$ | 111 | 0,51 | Toluol:Ethanol = 10:1 |
| 29c | H | $CH_3$ | $C_6H_5$ | H | $CH_2CH_2COOH$ | 95 | 0,34 | Toluol:Ethanol = 6:1 |
| 30a | H | H | 4-$H_3C$-$C_6H_5$ | $CH_3$ | H | 143 | 0,64 | Toluol:Ethanol = 3:1 |
| 30b | H | $CH_2CH_2CN$ | 4-$H_3C$-$C_6H_5$ | $CH_3$ | $CH_2CH_2CN$ | 98 | 0,21 | Toluol:Essigester = 3:1 |
| 30c | H | H | 4-$H_3C$-$C_6H_5$ | $CH_3$ | $CH_2CH_2COOH \cdot NEt_3$ | 119 | 0,57 | $CH_2Cl_2$:Methanol = 3:1 |

| Bsp. Nr. | $R^1$ | $R^2$ | $R^3$ | $R^4$ | Z | Schmelzpunkt (°C) | Rf-Wert | in |
|---|---|---|---|---|---|---|---|---|
| 31a | H | H | 1-$H_3C$-Indol-2-on-5-yl | H | H | 202 | 0,48 | Toluol:Ethanol = 3:1 |
| 31b | H | $CH_2CH_2CN$ | 1-$H_3C$-Indol-2-on-5-yl | H | $CH_2CH_2CN$ | Öl | 0,5 | Toluol:Ethanol = 3:1 |
| 31c | H | H | 1-$H_3C$-Indol-2-on-5-yl | H | $CH_2CH_2COOH$ | | | |
| 32a | H | H | Benzothiadiazol-4-yl | H | H | 155 | 0,5 | Toluol:Ethanol = 3:1 |
| 32b | H | $CH_2CH_2CN$ | Benzothiadiazol-4-yl | H | $CH_2CH_2CN$ | Öl | 0,6 | Toluol:Ethanol = 3:1 |
| 32c | H | H | 2,3-Di-$H_2N$-$C_6H_3$ | H | $CH_2CH_2COOH \cdot NEt_3$ | 121-7 | | |
| 33a | H | H | 4-$H_5C_2$-$C_6H_5$ | $CH_3$ | H | 111 | 0,63 | Toluol:Ethanol = 3:1 |
| 33b | H | $CH_2CH_2CN$ | 4-$H_5C_2$-$C_6H_5$ | $CH_3$ | $CH_2CH_2CN$ | 106 | 0,29 | Toluol:Essigester = 3:1 |
| 33c | H | H | 4-$H_5C_2$-$C_6H_5$ | $CH_3$ | $CH_2CH_2COOH$ | | | |
| 34a | H | H | 2-Thienyl | $CH_3$ | H | 127 | 0,54 | Toluol:Ethanol = 3:1 |
| 34b | H | $CH_2CH_2CN$ | 2-Thienyl | $CH_3$ | $CH_2CH_2CN$ | 139 | 0,24 | Toluol:Essigester = 3:1 |
| 34c | H | H | 2-Thienyl | $CH_3$ | $CH_2CH_2COOH \cdot NEt_3$ | 122 | 0,57 | $CHCl_3$:Methanol = 3:1 |
| 35a | H | H | 4-$H_3C$-$C_6H_4$ | $COCH_3$ | H | 167 | 0,2 | Toluol:Essigester = 3:1 |
| 35b | H | $CH_2CH_2CN$ | 4-$H_3C$-$C_6H_4$ | $COCH_3$ | $CH_2CH_2CN$ | 146 | 0,21 | Toluol:Essigester = 3:1 |
| 35c | H | H | 4-$H_3C$-$C_6H_4$ | $COCH_3$ | $CH_2CH_2COOH$ | | | |
| 36a | H | H | 4-Cl-$C_6H_4$ | $CH_3$ | H | 133 | 0,63 | Toluol:Ethanol = 3:1 |
| 36b | H | $CH_2CH_2CN$ | 4-Cl-$C_6H_4$ | $CH_3$ | $CH_2CH_2CN$ | 112 | 0,24 | Toluol:Essigester = 3:1 |
| 36c | H | H | 4-Cl-$C_6H_4$ | $CH_3$ | $CH_2CH_2COOH \cdot NEt_3$ | 123 | 0,6 | $CHCl_3$:Methanol = 3:1 |
| 37a | H | $CH_2CH(CH_3)CN$ | 4-Cl-$C_6H_4$ | H | $CH_2CH(CH_3)CN$ | 99 | 0,44 | Toluol:Ethanol = 10:1 |
| 37b | H | H | 4-Cl-$C_6H_4$ | H | $CH_2CH(CH_3)COOH$ | | 0,41 | Toluol:Ethanol = 3:1 |
| 38a | H | $CH_2CH(CH_3)CN$ | 4-Cl-$C_6H_4$ | $CH_3$ | $CH_2CH(CH_3)CN$ | Öl | 0,47 | Toluol:Aceton = 4:1 |
| 38b | H | H | 4-Cl-$C_6H_4$ | $CH_3$ | $CH_2CH(CH_3)COOH$ | | | |
| 39a | H | H | 4-n-$H_7C_3$-$C_6H_4$ | $CH_3$ | H | 118 | 0,44 | Toluol:Essigester = 3:1 |
| 39b | H | $CH_2CH_2CN$ | 4-n-$H_7C_3$-$C_6H_4$ | $CH_3$ | $CH_2CH_2CN$ | 112 | 0,28 | Toluol:Essigester = 3:1 |
| 39c | H | H | 4-n-$H_7C_3$-$C_6H_4$ | $CH_3$ | $CH_2CH_2COOH$ | | 0,28 | Toluol:Ethanol = 3:1 |

| Bsp. Nr. | $R^1$ | $R^2$ | $R^3$ | $R^4$ | Z | Schmelzpunkt (°C) | Rf-Wert in |
|---|---|---|---|---|---|---|---|
| 40a | H | H | 4-F-$C_6H_4$ | $CH_3$ | H | 132 | 0,65 Toluol:Ethanol = 3:1 |
| 40b | H | $CH_2CH_2CN$ | 4-F-$C_6H_4$ | $CH_3$ | $CH_2CH_2CN$ | 87 | 0,27 Toluol:Essigester = 3:1 |
| 40c | H | H | 4-F-$C_6H_4$ | $CH_3$ | $CH_2CH_2COOH$ | 111 | 0,51 $CHCl_3$:Methanol = 3:1 |
| 41a | H | H | 4-$H_3C$-$C_6H_4$ | $CH(OH)CH_3$ | H | 148 | 0,09 Toluol:Essigester = 3:1 |
| 41b | H | $CH_2CH_2CN$ | 4-$H_3C$-$C_6H_4$ | $CH(OH)CH_3$ | $CH_2CH_2CN$ | | |
| 41c | H | H | 4-$H_3C$-$C_6H_4$ | $CH(OH)CH_3$ | $CH_2CH_2COOH$ | | |
| 42a | H | H | $C_6H_5$ | $CH(CH_3)_2$ | H | | |
| 42b | H | $CH_2CH_2CN$ | $C_6H_5$ | $CH(CH_3)_2$ | $CH_2CH_2CN$ | | |
| 42c | H | H | $C_6H_5$ | $CH(CH_3)_2$ | $CH_2CH_2COOH$ | | |
| 43a | H | H | 4-Cl-$C_6H_4$ | $CH(CH_3)_2$ | H | | |
| 43b | H | $CH_2CH_2CN$ | 4-Cl-$C_6H_4$ | $CH(CH_3)_2$ | $CH_2CH_2CN$ | | |
| 43c | H | H | 4-Cl-$C_6H_4$ | $CH(CH_3)_2$ | $CH_2CH_2COOH$ | | |
| 44a | H | H | 4-F-$C_6H_4$ | $CH(CH_3)_2$ | H | | |
| 44b | H | $CH_2CH_2CN$ | 4-F-$C_6H_4$ | $CH(CH_3)_2$ | $CH_2CH_2CN$ | | |
| 44c | H | H | 4-F-$C_6H_4$ | $CH/CH_3)_2$ | $CH_2CH_2COOH$ | | |
| 45a | H | H | $C_6H_5$ | $C(CH_3)_3$ | H | | |
| 45b | H | $CH_2CH_2CN$ | $C_6H_5$ | $C(CH_3)_3$ | $CH_2CH_2CN$ | | |
| 45c | H | H | $C_6H_5$ | $C(CH_3)_3$ | $CH_2CH_2COOH$ | | |
| 46a | H | H | 4-Cl-$C_6H_4$ | $C(CH_3)_3$ | H | | |
| 46b | H | $CH_2CH_2CN$ | 4-Cl-$C_6H_4$ | $C(CH_3)_3$ | $CH_2CH_2CN$ | | |
| 46c | H | H | 4-Cl-$C_6H_4$ | $C(CH_3)_3$ | $CH_2CH_2COOH$ | | |
| 47a | H | H | 4-F-$C_6H_4$ | $C(CH_3)_3$ | H | | |
| 47b | H | $CH_2CH_2CN$ | 4-F-$C_6H_4$ | $C(CH_3)_3$ | $CH_2CH_2CN$ | | |
| 47c | H | H | 4-F-$C_6H_4$ | $C(CH_3)_3$ | $CH_2CH_2COOH$ | | |

| Bsp. Nr. | $R^1$ | $R^2$ | $R^3$ | $R^4$ | Z | Schmelzpunkt (°C) | Rf-Wert in |
|---|---|---|---|---|---|---|---|
| 48a | 5-F | H | 4-F-$C_6H_5$ | $CH_3$ | H | 162-163 | 0,32 Toluen:Ethylacetat = 8:2 |
| 48b | 5-F | $CH_2CH_2CN$ | 4-F-$C_6H_5$ | $CH_3$ | $CH_2CH_2CN$ | Öl | 0,25 Toluen:Ethylacetat = 8:2 |
| 48c | 5-F | H | 4-F-$C_6H_5$ | $CH_3$ | $CH_2CH_2COOH$ | 169-172 | 0,5 $CH_2Cl_2$:Methanol = 9:1 |
| 49a | 6-F | H | 4-F-$C_6H_5$ | $CH_3$ | H | 142-143 | 0,42 Toluen:Ethylacetat = 8:2 |
| 49b | 6-F | $CH_2CH_2CN$ | 4-F-$C_6H_5$ | $CH_3$ | $CHCH_2CN$ | Öl | 0,29 Toluen:Ethylacetat = 8:2 |
| 49c | 6-F | H | 4-F-$C_6H_5$ | $CH_3$ | $CH_2CH_2COOH$ | Öl | 0,47 $CH_2Cl_2$:Methanol = 9:1 |
| 50a | 4-F | H | 4-F-$C_6H_5$ | $CH_3$ | H | 155-156 | 0,42 Toluen:Ethylacetat = 8:2 |
| 50b | 4-F | $CH_2CH_2CN$ | 4-F-$C_6H_5$ | $CH_3$ | $CHCH_2CN$ | Öl | 0,34 Toluen:Ethylacetat = 8:2 |
| 50c | 4-F | H | 4-F-$C_6H_5$ | $CH_3$ | $CH_2CH_2COOH$ | Öl | 0,37 $CH_2Cl_2$:Methanol = 9:1 |
| 51a | 7-F | H | 4-F-$C_6H_5$ | $CH_3$ | H | Öl | 0,32 Toluen:Ethylacetat = 8:2 |
| 51b | 7-F | $CH_2CH_2CN$ | 4-F-$C_6H_5$ | $CH_3$ | $CH_2CH_2CN$ | Öl | 0,25 Toluen:Ethylacetat = 8:2 |
| 51c | 7-F | H | 4-F-$C_6H_5$ | $CH_3$ | $CH_2CH_2COOH$ | Öl | 0,72 $CH_2Cl_2$:Methanol = 9:1 |
| 52a | 5-$OCH_3$ | H | 4-F-$C_6H_5$ | $CH_3$ | H | Öl | 0,3 Toluen:Ethylacetat = 8:2 |
| 52b | 5-$OCH_3$ | $CH_2CH_2CN$ | 4-F-$C_6H_5$ | $CH_3$ | $CH_2CH_2CN$ | Öl | 0,18 Toluen:Ethylacetat = 8:2 |
| 52c | 5-$OCH_3$ | H | 4-F-$C_6H_5$ | $CH_3$ | $CH_2CH_2COOH$ | Öl | 0,45 $CH_2Cl_2$:Methanol = 9:1 |
| 53a | 4-$CH_3$ | H | 4-F-$C_6H_5$ | $CH_3$ | H | Öl | 0,4 Toluen:Ethylacetat = 8:2 |
| 53b | 4-$CH_3$ | $CH_2CH_2CN$ | 4-F-$C_6H_5$ | $CH_3$ | $CH_2CH_2CN$ | Öl | 0,24 Toluen:Ethylacetat = 8:2 |
| 53c | 4-$CH_3$ | H | 4-F-$C_6H_5$ | $CH_3$ | $CH_2CH_2COOH$ | Öl | 0,62 $CH_2Cl_2$:Methanol = 9:1 |

Anwendungsbeispiel

Beispiel 25

Zur Bestimmung der thrombozytenaggregationshemmenden Wirkung wurde Blut von gesunden Probanden beiderlei Geschlechts verwendet. Als Antikoagulans wurden einem Teil 3,8 %iger wäßriger Natriumzitratlösung 9 Teile Blut zugemischt. Mittels Zentrifugation erhält man aus diesem Blut plättchenreiches Zitratplasma (PRP) (Jürgens/Beller, Klinische Methoden der Blutgerinnungsanalyse; Thieme Verlag, Stuttgart 1959).

Für diese Untersuchungen wurden 0,8 ml PRP und 0,1 ml der Wirkstofflösung bei 37°C im Wasserbad vorinkubiert. Anschließend wurde die Thrombozytenaggregation nach der turbidometrischen Methode (Born, B.V.R., J. Physiol. (London), 162, 67, 1962) im Aggregometer bei 37°C bestimmt (Therapeutische Berichte 47, 80-86, 1975). Hierzu wurde die vorinkubierte Probe mit 0,1 ml Kollagen, einem aggregationsauslösenden Agens, versetzt. Die Veränderung der optischen Dichte in der Probe des PRP wurde während einer Zeitdauer von 6 Minuten aufgezeichnet und der Ausschlag nach 6 Minuten bestimmt. Hierzu wird die prozentuale Hemmung gegenüber der Kontrolle errechnet.

| N-Indolylethyl-sulfonsäureamid nach Beispiel-Nr. | Grenzkonzentration für Hemmung (mg/l) |
|---|---|
| 1c | 0,3-0,03 |
| 4b | 0,3-0,03 |
| 5b | 0,3-1,0 |
| 7c | 0,3-0,03 |
| 9c | 10-3 |
| 14c | 10-3 |
| 19c | 10-3 |

**Patentansprüche**

1. N-Indolylethyl-sulfonsäureamide der Formel

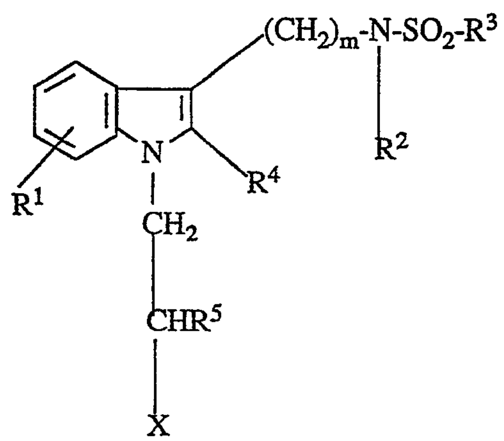

(I)

in der

$R^1$ Wasserstoff, Fluor, Chlor, Brom, Trifluormethyl, Carboxy, Niederalkoxy-($C_1$ bis $C_6$)carbonyl, die Gruppe

$$-S(O)_nR^6$$

in der

$R^6$ Niederalkyl ($C_1$ bis $C_6$) oder Phenyl und

n eine der Zahlen 0, 1 oder 2 bedeutet,
die Gruppe

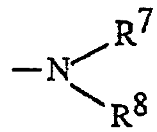

in der
$R^7$ und $R^8$ gleich oder verschieden sind und Wasserstoff, Niederalkyl ($C_1$ bis $C_6$), Phenyl, Benzyl oder Acetyl bedeuten, oder
die Gruppe

$$-O-R^9$$

in der
$R^9$ Wasserstoff, Niederalkyl ($C_1$ bis $C_6$), Phenyl, $C_7$ bis $C_{10}$-Aralkyl, $-SO_2$-Niederalkyl ($C_1$ bis $C_6$), $-SO_2$-Phenyl, $-SO_2$-Benzyl oder Trifluormethyl bedeutet, oder
gegebenenfalls durch Carboxy, Niederalkoxy ($C_1$ bis $C_6$)carbonyl, Fluor, Chlor, Brom und/oder Cyano substituiertes Niederalkyl ($C_1$ bis $C_6$), Niederalkenyl ($C_2$ bis $C_6$), Cyclopentyl oder Cyclohexyl bedeutet,
$R^2$ Wasserstoff bedeutet,
$R^3$ Niederalkyl ($C_1$ bis $C_6$), Niederalkenyl ($C_2$ bis $C_6$), Cyclopentyl, Cyclohexyl, gegebenenfalls bis zu vierfach durch Fluor, Chlor, Brom, Cyano, Hydroxy, Trifluormethyl, Trifluormethoxy, Niederalkyl($C_1$ bis $C_6$), Niederalkenyl($C_2$ bis $C_6$), die selbst wiederum durch Carboxy oder $C_1$ bis $C_4$-Alkoxycarbonyl substituiert sein können, Niederalkoxy ($C_1$ bis $C_6$), Carboxyl, $C_1$ bis $C_4$-Alkoxycarboxyl, Benzyloxy, Benzylthio, Phenyl, Phenoxy und/oder die Gruppe

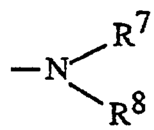

in der
$R^7$ und $R^8$ die obengenannte Bedeutung haben,
gegebenenfalls substituiertes Phenyl oder Naphthyl, oder
gegebenenfalls bis zu vierfach durch Fluor, Chlor, Brom, Amino, Dimethylamino, Acetylamino, Trifluormethyl, Trifluormethoxy, $C_1$ bis $C_4$-Alkyl und/oder $C_1$ bis $C_4$-Alkoxy substituiertes Pyridyl, Thienyl, Furyl, Pyrimidyl, Piperazinyl, Piperidinyl, Morpholinyl, Thiomorpholinyl, Chinolyl, Isochinolyl, Imidazolyl, Triazolyl, Tetrazolyl, Thiadiazolyl, Pyrrolyl, Benzimidazolyl, Benzothiadiazolyl, Indolyl, Indolonyl, Thiazolyl, Isothiazolyl, Benzthiazolyl, Benzoisothiazolyl, Benzoisothiazolonyl bedeutet,
$R^4$ Wasserstoff, Niederalkyl ($C_1$ bis $C_6$), Niederhydroxyalkyl ($C_1$ bis $C_6$), Niederalkenyl($C_2$ bis $C_6$), Niederalkyl($C_1$ bis $C_6$)carbonyl, Cyano, gegebenenfalls bis zu dreifach durch Fluor, Chlor, Methyl, Methoxy und/oder Trifluormethyl substituiertes Phenyl, Pyridyl, Thienyl, Furyl, Pyrimidyl, Imidazolyl oder Pyrrolyl bedeutet,
$R^5$ Wasserstoff oder Niederalkyl($C_1$ bis $C_6$) bedeutet,
X Carboxy bedeutet und
m die Zahl 2 bedeutet,
und deren Salze.

2. N-Indolylethyl-sulfonsäureamide nach Anspruch 1, wobei
$R^1$ Wasserstoff, Fluor, Chlor, Brom, Trifluormethyl, die Gruppe

$$-S(O)_n-R^6$$

in der
$R^6$ Methyl, Ethyl oder Phenyl und
n eine der Zahlen 0 oder 2 bedeutet,
Amino, Methylamino, Dimethylamino, Acetylamino, die Gruppe

$$-O-R^9$$

in der
$R^9$ Wasserstoff, $C_1$ bis $C_4$-Alkyl, Phenyl oder Benzyl bedeutet, oder
gegebenenfalls durch Fluor, Chlor und/oder Cyano substituiertes $C_1$ bis $C_4$-Alkyl bedeutet,
$R^2$ Wasserstoff bedeutet,
$R^3$ $C_1$ bis $C_4$-Alkyl, gegebenenfalls bis zu dreifach durch Fluor, Chlor, Brom, Cyano, Hydroxy, Trifluormethyl, Carboxyvinyl, $C_1$ bis $C_4$-Alkyl, $C_1$ bis $C_4$-Alkoxy, $C_1$ bis $C_4$-Meth- oder Ethoxycarbonylalkyl, Phenyl, Phenoxy, Amino, Dimethylamino und/oder Acetylamino substituiertes Phenyl oder Naphthyl, oder durch $C_1$ bis $C_4$-Alkyl, Fluor, Chlor, Amino, Dimethylamino substituiertes Pyridyl, Thienyl, Furyl,

Pyrimidyl, Piperazinyl, Piperidinyl, Morphonlinyl oder Chinolyl, Benzoisothiazolonyl, Benzothiazolyl oder Indolonyl bedeutet,
$R^4$ Wasserstoff, Methyl, Ethyl, Hydroxymethyl, Hydroxyethyl, Methylcarbonyl, Cyano oder Phenyl bedeutet,
$R^5$ Wasserstoff oder $C_1$ bis $C_4$-Alkyl bedeutet,
X Carboxy bedeutet, und
m für 2 steht,
und deren Salze.

3. Zwischenprodukte der Formel

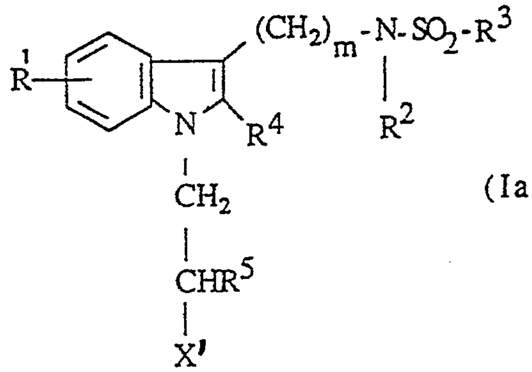

(Ia)

in welcher
$R^1$ Wasserstoff, Fluor, Chlor, Brom, Trifluormethyl, Carboxy, Niederalkoxy($C_1$ bis $C_6$)carbonyl, die Gruppe

$$-S(O)_n-R^6$$

in der
$R^6$ Niederalkyl ($C_1$ bis $C_6$) oder Phenyl und
n eine der Zahlen 0, 1 oder 2 bedeutet,
die Gruppe

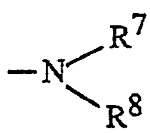

in der
$R^7$ und $R^8$ gleich oder verschieden sind und Wasserstoff, Niederalkyl ($C_1$ bis $C_6$), Phenyl, Benzyl oder Acetyl bedeuten, oder
die Gruppe

$$-O-R^9$$

in der
$R^9$ Wasserstoff, Niederalkyl ($C_1$ bis $C_6$), Phenyl, $C_7$ bis $C_{10}$-Aralkyl–$SO_2$-Niederalkyl ($C_1$ bis $C_6$), –$SO_2$-Phenyl, –$SO_2$-Benzyl oder Trifluormethyl bedeutet, oder
gegebenenfalls durch Carboxy, Niederalkoxy($C_1$ bis $C_6$)carbonyl, Fluor, Chlor, Brom und/oder Cyano substituiertes Niederalkyl ($C_1$ bis $C_6$), Niederalkenyl ($C_2$ bis $C_6$), Cyclopentyl oder Cyclohexyl bedeutet,
$R^2$ gegebenenfalls durch Fluor, Chlor, Brom, Carboxy, Niederalkoxy ($C_1$ bis $C_6$)carbonyl und/oder Cyano substituiertes Niederalkyl ($C_1$ bis $C_6$), Niederalkenyl ($C_1$ bis $C_6$), Cyclopentyl oder Cyclohexyl bedeutet,
$R^3$ Niederalkyl ($C_1$ bis $C_6$), Niederalkenyl ($C_2$ bis $C_6$), Cyclopentyl, Cyclohexyl, gegebenenfalls bis zu vierfach durch Fluor, Chlor, Brom, Cyano, Hydroxy, Trifluormethyl, Trifluormethoxy, Niederalkyl ($C_1$ bis $C_6$), Niederalkenyl ($C_1$ bis $C_6$), die selbst wiederum durch Carboxy oder $C_1$ bis $C_4$-Alkoxycarbonyl substituiert sein können, Niederalkoxy ($C_1$ bis $C_6$), Carboxyl, $C_1$ bis $C_4$-Alkoxycarboxyl, Benzyloxy, Benzylthio, Phenyl, Phenoxy und/oder die Gruppe

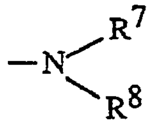

in der
$R^7$ und $R^8$ die obengenannte Bedeutung haben,
gegebenenfalls substituiertes Phenyl oder Naphthyl, oder

gegebenenfalls bis zu vierfach durch Fluor, Chlor, Brom, Amino, Dimethylamino, Acetylamino, Trifluormethyl, Trifluormethoxy, $C_1$ bis $C_4$-Alkyl und/oder $C_1$ bis $C_4$-Alkoxy substituiertes Pyridyl, Thienyl, Furyl, Pyrimidyl, Piperazinyl, Piperidinyl, Morpholinyl, Thiomorpholinyl, Chinolyl, Isochinolyl, Imidazolyl, Triazolyl, Tetrazolyl, Thiadiazolyl, Pyrrolyl, Benzimidazolyl, Benzothiadiazolyl, Indolyl, Indolonyl, Thiazolyl, Isothiazolyl, Benzthiazolyl, Benzoisothiazolyl, Benzoisothiazolonyl bedeutet,
$R^4$ Wasserstoff, Niederalkyl ($C_1$ bis $C_6$), Niederhydroxyalkyl ($C_1$ bis $C_6$), Niederalkenyl($C_2$ bis $C_6$), Niederalkyl($C_1$ bis $C_6$)carbonyl, Cyano, gegebenenfalls bis zu dreifach durch Fluor, Chlor, Methyl, Methoxy und/oder Trifluormethyl substituiertes Phenyl, Pyridyl, Thienyl, Furyl, Pyrimidyl, Imidazolyl oder Pyrrolyl bedeutet,
$R^5$ Wasserstoff oder Niederalkyl($C_1$ bis $C_6$) bedeutet,
X′ Niederalkoxy($C_1$ bis $C_6$)carbonyl oder Cyano bedeutet und
m die Zahl 2 bedeutet
und deren Salze.

4. Verfahren zur Herstellung von N-Indolylethylsulfonamiden und deren Salzen nach Anspruch 1, dadurch gekennzeichnet, daß man Indolalkylamine der Formel

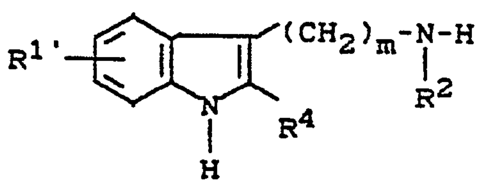

(II),

in welcher
$R^{1\prime}$ Wasserstoff, Fluor, Chlor, Brom, Trifluormethyl, Carboxy, Niederalkoxy($C_1$ bis $C_6$)carbonyl,
die Gruppe

$$-S(O)_n-R^6$$

in der
$R^6$ Niederalkyl ($C_1$ bis $C_6$) oder Phenyl und
n eine der Zahlen 0, 1 oder 2 bedeutet,
die Gruppe

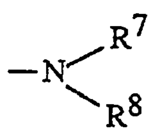

in der
$R^7$ und $R^8$ gleich oder verschieden sind und Wasserstoff, Niederalkyl($C_1$ bis $C_6$), Phenyl, Benzyl oder Acetyl bedeuten, oder
die Gruppe

$$-O-R^9$$

in der
$R^9$ Niederalkyl ($C_1$ bis $C_6$), Phenyl, $C_7$ bis $C_{10}$-Aralkyl$-SO_2$-Niederalkyl ($C_1$ bis $C_6$), $-SO_2$-Phenyl, $-SO_2$-Benzyl oder Trifluormethyl bedeutet,
oder gegebenenfalls durch Carboxy, Niederalkoxy($C_1$ bis $C_6$)carbonyl, Fluor, Chlor, Brom und/oder Cyano substituiertes Niederalkyl($C_1$ bis $C_6$), Niederalkenyl($C_1$ bis $C_6$), Cyclopentyl oder Cyclohexyl bedeutet,
$R^2$ Wasserstoff bedeutet,
$R^4$ Wasserstoff, Niederalkyl ($C_1$ bis $C_6$), Niederhydroxyalkyl ($C_1$ bis $C_6$), Niederalkenyl($C_2$ bis $C_6$), Niederalkyl($C_1$ bis $C_6$)carbonyl, Cyano, gegebenenfalls bis zu dreifach durch Fluor, Chlor, Methyl, Methoxy und/oder Trifluormethyl substituiertes Phenyl, Pyridyl, Thienyl, Furyl, Pyrimidyl, Imidazolyl oder Pyrrolyl bedeutet, und
m die Zahl 2 bedeutet,
mit Sulfonsäurehalogeniden der Formel

$$R^3-SO_2-Y \qquad (III),$$

in der
$R^3$ Niederalkyl ($C_1$ bis $C_6$), Niederalkenyl ($C_2$ bis $C_6$), Cyclopentyl, Cyclohexyl, gegebenenfalls bis zu vierfach durch Fluor, Chlor, Brom, Cyano, Hydroxy, Trifluormethyl, Trifluormethoxy, Niederalkyl($C_1$ bis $C_6$), Niederalkenyl ($C_2$ bis $C_6$), die selbst wiederum durch Carboxy oder $C_1$ bis $C_4$-Alkoxycarbonyl substituiert sein können, Niederalkoxy ($C_1$ bis $C_6$), Carboxyl, $C_1$ bis $C_4$-Alkoxycarboxyl, Benzyloxy, Benzylthio, Phenyl, Phenoxy und/oder die Gruppe

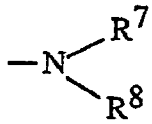

in der
$R^7$ und $R^8$ die obengenannte Bedeutung haben,
gegebenenfalls substituiertes Phenyl oder Naphthyl, oder
gegebenenfalls bis zu vierfach durch Fluor, Chlor, Brom, Amino, Dimethylamino, Acetylamino, Trifluormethyl, Trifluormethoxy, $C_1$ bis $C_4$-Alkyl und/oder $C_1$ bis $C_4$-Alkoxy substituiertes Pyridyl, Thienyl, Furyl, Pyrimidyl, Piperazinyl, Piperidinyl, Morpholinyl, Thiomorpholinyl, Chinolyl, Isochinolyl, Imidazolyl, Triazolyl, Tetrazolyl, Thiadiazolyl, Pyrrolyl, Benzimidazolyl, Benzothiadiazolyl, Indolyl, Indolonyl, Thiazolyl, Isothiazolyl, Benzthiazolyl, Benzoisothiazolyl, Benzoisothiazolonyl bedeutet, und
Y für Halogen steht,
in Gegenwart eines inerten Lösungsmittels, gegebenenfalls in Gegenwart einer Base, umsetzt, dann mit einer olefinischen Verbindung der Formel

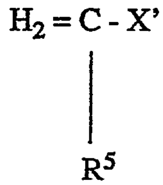

(IV),

in der
X' Niederalkoxy($C_1$ bis $C_6$)carbonyl oder Cyano bedeutet und
$R^5$ Wasserstoff oder Niederalkyl($C_1$ bis $C_6$) bedeutet,
in Gegenwart eines inerten Lösungsmittels, gegebenenfalls in Gegenwart einer Base zu den Zwischenprodukten der Formel (Ia) alkyliert, dann für den Fall der Herstellung von in 4-, 5-, 6- oder 7-Stellung durch Hydroxy substituierten Verbindungen die entsprechenden Benzyloxyverbindungen in Gegenwart eines Katalysators in einem inerten Lösungsmittel, gegebenenfalls in Gegenwart einer Säure hydriert, dann für den Fall der Herstellung von N-Indolylethylcarbonyl-Verbindungen die entsprechenden Nitrile oder Alkoxycarbonyle verseift,
und dann für den Fall der Herstellung von Salzen mit einer entsprechenden Base umsetzt.

5. Verfahren nach Anspruch 4, dadurch gekennzeichnet, daß es im Temperaturbereich von –20 bis 100°C durchgeführt wird.

6. Verfahren nach den Ansprüchen 4 und 5, dadurch gekennzeichnet, daß bei der Herstellung der Hydroxyverbindungen die Hydrierung in Gegenwart eines Edelmetallkatalysators durchgeführt wird.

7. Verfahren nach den Ansprüchen 4 und 5, dadurch gekennzeichnet, daß bei der Herstellung der Carboxylverbindungen die Verseifung in Gegenwart eines Alkali- oder Erdalkali-hydroxylats oder -alkoholats durchgeführt wird.

8. Arzneimittel, enthaltend eines oder mehrere 4-Indolyl-ethyl-sulfonsäureamide nach den Ansprüchen 1 bis 2.

9. Verwendung von N-Indolyl-ethyl-sulfonsäureamiden nach den Ansprüchen 1 bis 2 zur Herstellung von Arzneimitteln.

10. Verwendung von N-Indolyl-ethyl-sulfonsäureamiden nach den Ansprüchen 1 bis 2 zur Herstellung von Arzneimitteln für die Bekämpfung und Behandlung von Thrombosen, Thromboembolien, Ischämien, Allergien und asthmatischen Störungen.

## Claims

1. N-Indolylethyl-sulphonic acid amides of the formula

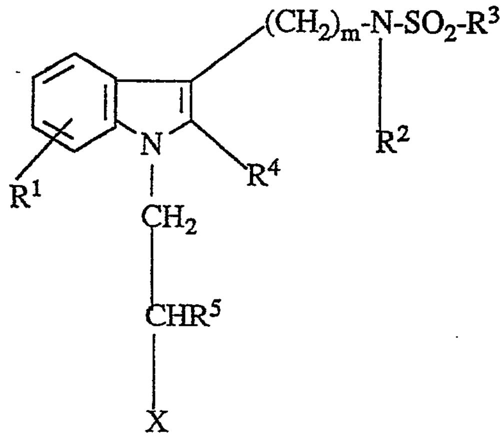

(I)

in which
$R^1$ denotes hydrogen, fluorine, chlorine, bromine, trifluoromethyl, carboxyl, lower alkoxy($C_1$ to $C_6$)-carbonyl, the group

$$-S(O)_nR^6$$

in which
$R^6$ denotes lower alkyl($C_1$ to $C_6$) or phenyl and
n denotes one of the numbers, 0, 1 or 2,
the grup

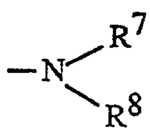

in which
$R^7$ and $R^8$ are identical or different and denote hydrogen, lower alkyl($C_1$ to $C_6$), phenyl, benzyl or acetyl, or the group

$$-O-R^9$$

in which
$R^9$ denotes hydrogen, lower alkyl($C_1$ to $C_6$), phenyl, $C_7$ to $C_{10}$-aralkyl, $-SO_2$-lower alkyl($C_1$ to $C_6$), $-SO_2$-phenyl, $-SO_2$-benzyl or trifluoromethyl, or lower alkyl($C_1$ to $C_6$), lower alkenyl ($C_2$ to $C_6$), cyclopentyl or cyclohexyl, optionally substituted by carboxyl, lower alkoxy($C_1$ to $C_6$)carbonyl, fluorine, chlorine, bromine and/or cyano,
$R^2$ denotes hydrogen,
$R^3$ denotes lower alkyl($C_1$ to $C_6$), lower alkenyl($C_2$ to $C_6$), cyclopentyl or cyclohexyl, phenyl or naphthyl which is optionally substituted by up to four substituents from the group comprising fluorine, chlorine, bromine, cyano, hydroxyl, trifluoromethyl, trifluoromethoxy, lower alkyl($C_1$ to $C_6$) or lower alkenyl($C_2$ to $C_6$), which can themselves in turn be substituted by carboxyl or $C_1$ to $C_4$-alkoxy-carbonyl, lower alkoxy($C_1$ to $C_6$), carboxyl, $C_1$ to $C_4$-alkoxy-carboxyl, benzyloxy, benzylthio, phenyl, phenoxy and/or the group

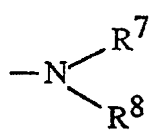

in which
$R^7$ and $R^8$ have the abovementioned meaning, or pyridyl, thienyl, furyl, pyrimidyl, piperazinyl, piperidinyl, morpholinyl, thiomorpholinyl, quinolyl, isoquinolyl, imidazolyl, triazolyl, tetrazolyl, thiadiazolyl, pyrrolyl, benzimidazolyl, benzothiadiazolyl, indolyl, indolonyl, thiazolyl, isothiazolyl, benzothiazolyl, benzoisothiazolyl or benzoisothiazolonyl, optionally substituted by up to four substituents from the group comprising fluorine, chlorine, bromine, amino, dimethylamino, acetylamino, trifluoromethyl, trifluoromethoxy, $C_1$ to $C_4$-alkyl and/or $C_1$ to $C_4$-alkoxy,
$R^4$ denotes hydrogen, lower alkyl($C_1$ to $C_6$), lower hydroxyalkyl($C_1$ to $C_6$), lower alkenyl($C_2$ to $C_6$), lower

alkyl($C_1$ to $C_6$)carbonyl, cyano, or phenyl, pyridyl, thienyl, furyl, pyrimidyl, imidazolyl or pyrrolyl optionally substituted by up to three substituents from the group comprising fluorine, chlorine, methyl, methoxy and/or trifluoromethyl,
$R^5$ denotes hydrogen or lower alkyl($C_1$ to $C_6$),
X denotes carboxyl and
m denotes the number 2
and salts thereof.

2. N-Indolylethyl-sulphonic acid amides according to Claim 1, wherein
$R^1$ denotes hydrogen, fluorine, chlorine, bromine, trifluoromethyl, the group

$$-S(O)_n-R^6$$

in which
$R^6$ denotes methyl, ethyl or phenyl and
n denotes one of the numbers 0 or 2, amino, methylamino, dimethylamino, acetylamino, the group

$$-O-R^9$$

in which
$R^9$ denotes hydrogen, $C_1$ to $C_4$-alkyl, phenyl or benzyl, or $C_1$ to $C_4$-alkyl, optionally substituted by fluorine, chlorine and/or cyano,
$R^2$ denotes hydrogen,
$R^3$ denotes $C_1$ to $C_4$-alkyl, phenyl or naphthyl, optionally substituted by up to three substituents from the group comprising fluorine, chlorine, bromine, cyano, hydroxyl, trifluoromethyl, carboxyvinyl, $C_1$ to $C_4$-alkyl, $C_1$ to $C_4$-alkoxy, $C_1$ to $C_4$-meth- or ethoxycarbonylalkyl, phenyl, phenoxy, amino, dimethylamino and/or acetylamino, or pyridyl, thienyl, furyl, pyrimidyl, piperazinyl, piperidinyl, morpholinyl or quinolyl, benzoisothiazolonyl, benzothiazolyl or indolonyl, substituted by $C_1$ to $C_4$-alkyl, fluorine, chlorine, amino or dimethylamino,
$R^4$ denotes hydrogen, methyl, ethyl, hydroxymethyl, hydroxyethyl, methylcarbonyl, cyano or phenyl,
$R^5$ denotes hydrogen or $C_1$ to $C_4$-alkyl,
X denotes carboxyl and
m represents 2,
and salts thereof.

3. Intermediates of the formula

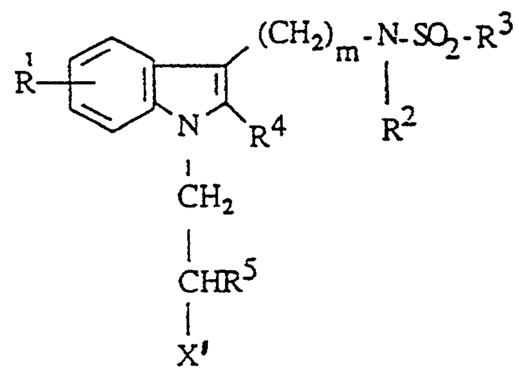

in which
$R^1$ denotes hydrogen, fluorine, chlorine, bromine, trifluoromethyl, carboxyl, lower alkoxy($C_1$ to $C_6$)-carbonyl, the group

$$-S(O)_n-R^6$$

in which
$R^6$ denotes lower alkyl($C_1$ to $C_6$) or phenyl and
n denotes one of the numbers 0, 1 or 2,
the group

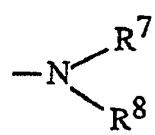

in which
$R^7$ and $R^8$ are identical or different and denote hydrogen, lower alkyl($C_1$ to $C_6$), phenyl, benzyl, or acetyl, or the group

$-O-R^9$

in which

$R^9$ denotes hydrogen, lower alkyl($C_1$ to $C_6$), phenyl, $C_7$ to $C_{10}$-aralkyl, $-SO_2$-lower alkyl($C_1$ to $C_6$), $-SO_2$-phenyl, $-SO_2$-benzyl or trifluoromethyl, or lower alkyl($C_1$ to $C_6$), lower alkenyl($C_2$ to $C_6$), cyclopentyl or cyclohexyl, optionally substituted by carboxyl, lower alkoxy($C_1$ to $C_6$)carbonyl, fluorine, chlorine, bromine and/or cyano,

$R^2$ denotes lower alkyl($C_1$ to $C_6$), lower alkenyl($C_1$ to $C_6$), cyclopentyl or cyclohexyl, optionally substituted by fluorine, chlorine, bromine, carboxyl, lower alkoxy ($C_1$ to $C_6$) carbonyl and/or cyano,

$R^3$ denotes lower alkyl($C_1$ to $C_6$), lower alkenyl($C_2$ to $C_6$), cyclopentyl or cyclohexyl, phenyl or naphthyl which is optionally substituted by up to four substituents from the group comprising fluorine, chlorine, bromine, cyano, hydroxyl, trifluoromethyl, trifluoromethoxy, lower alkyl($C_1$ to $C_6$) or lower alkenyl($C_2$ to $C_6$), which can themselves in turn be substituted by carboxyl or $C_1$ to $C_4$-alkoxycarbonyl, lower alkoxy($C_1$ to $C_6$), carboxyl, $C_1$ to $C_4$-alkoxycarboxyl, benzyloxy, benzylthio, phenyl, phenoxy and/or the group

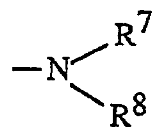

in which

$R^7$ and $R^8$ have the abovementioned meaning, or pyridyl, thienyl, furyl, pyrimidyl, piperazinyl, piperidinyl, morpholinyl, thiomorpholinyl, quinolyl, isoquinolyl, imidazolyl, triazolyl, tetrazolyl, thiadiazolyl, pyrrolyl, benzimidazolyl, benzothiadiazolyl, indolyl, indelonyl, thiazolyl, isothiazolyl, benzothiazolyl, benzoisothiazolyl or benzoisothiazolonyl, optionally substituted by up to four substituents from the group comprising fluorine, chlorine, bromine, amino, dimethylamino, acetylamino, trifluoromethyl, trifluoromethoxy, $C_1$ to $C_4$-alkyl and/or $C_1$ to $C_4$-alkoxy,

$R^4$ denotes hydrogen, lower alkyl($C_1$ to $C_6$), lower hydroxyalkyl($C_1$ to $C_6$), lower alkenyl($C_2$ to $C_6$), lower alkyl($C_1$ to $C_6$)carbonyl, cyano, or phenyl, pyridyl, thienyl, furyl, pyrimidyl, imidazolyl or pyrrolyl optionally substituted by up to three substituents from the group comprising fluorine, chlorine, methyl, methoxy and/or trifluoromethyl,

$R^5$ denotes hydrogen or lower alkyl($C_1$ to $C_6$),

X' denotes lower alkoxy($C_1$ to $C_6$)carbonyl or cyano and

m denotes the number 2

and salts thereof.

4. Process for the preparation of N-indolylethyl-sulphonamides and salts thereof according to Claim 1, characterized in that indolalkylamines of the formula

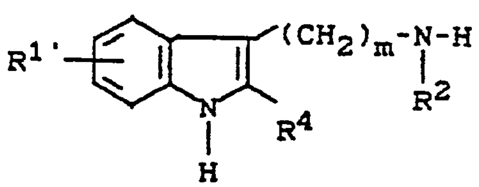

(II)

in which

$R^1$ denotes hydrogen, fluorine, chlorine, bromine, trifluoromethyl, carboxyl, lower alkoxy($C_1$ to $C_6$)-carbonyl, the group

$-S(O)_n-R^6$

in which

$R^6$ denotes lower alkyl($C_1$ to $C_6$) or phenyl and

n denotes one of the numbers, 0, 1 or 2,

or denotes the group

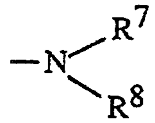

in which

$R^7$ and $R^8$ are identical or different and denote hydrogen, lower alkyl($C_1$ to $C_6$), phenyl, benzyl or acetyl, or the group

$-O-R^9$

in which

$R^9$ denotes lower alkyl($C_1$ to $C_6$), phenyl, $C_7$ to $C_{10}$-aralkyl$-SO_2$-lower alkyl($C_1$ to $C_6$), $-SO_2$-phe-

nyl, $-SO_2$-benzyl or trifluoromethyl, or lower alkyl($C_1$ to $C_6$), lower alkenyl($C_1$ to $C_6$), cyclopentyl or cyclohexyl, optionally substituted by carboxyl, lower alkoxy($C_1$ to $C_6$), fluorine, chlorine, bromine and/or cyano,
$R^2$ denotes hydrogen,
$R^4$ denotes hydrogen, lower alkyl ($C_1$ to $C_6$), lower hydroxyalkyl ($C_1$ to $C_6$), lower alkenyl ($C_2$ to $C_6$), lower alkyl ($C_1$ to $C_6$) carbonyl or cyano, or phenyl, pyridyl, thienyl, furyl, pyrimidyl, imidazolyl or pyrrolyl, optionally substituted by up to three substituents from the group comprising fluorine, chlorine, methyl, methoxy, and/or trifluoromethyl, and
m is the number 2,
are reacted with sulphonic acid halides of the formula

$$R_3–SO_2–Y \qquad (III),$$

in which
$R^3$ denotes lower alkyl($C_1$ to $C_6$), lower alkenyl($C_2$ to $C_6$), cyclopentyl or cyclohexyl, phenyl or naphthyl which is optionally substituted by up to four substituents from the group comprising fluorine, chlorine, bromine, cyano, hydroxyl, trifluoromethyl, trifluoromethoxy, lower alkyl($C_1$ to $C_6$) or lower alkenyl($C_2$ to $C_6$), which can themselves in turn be substituted by carboxyl or $C_1$ to $C_4$-alkoxycarbonyl, lower alkoxy($C_1$ to $C_6$), carboxyl, $C_1$ to $C_4$-alkoxycarboxyl, benzyloxy, benzylthio, phenyl, phenoxy and/or the group

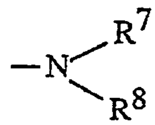

in which
$R^7$ and $R^8$ have the abovementioned meaning, or pyridyl, thienyl, furyl, pyrimidyl, piperazinyl, piperidinyl, morpholinyl, thiomorpholinyl, quinolyl, isoquinolyl, imidazolyl, triazolyl, tetrazolyl, thiadiazolyl, pyrrolyl, benzimidazolyl, benzothiadiazolyl, indolyl, indelonyl, thiazolyl, isothiazolyl, benzothiazolyl, benzoisithiazolyl or benzoisothiazolonyl, optionally substituted by up to four substituents from the group comprising fluorine, chlorine, bromine, amino, dimethylamino, acetylamino, trifluoromethyl, trifluoromethoxy, $C_1$ to $C_4$-alkyl and/or $C_1$ to $C_4$-alkoxy, and
Y represents halogen,
in the presence of an inert solvent, if appropriate in the presence of a base, and the product is then alkylated to give the intermediates of the formula (1a) with an olefinic compound of the formula

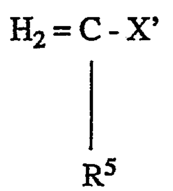

(IV)

in which
X′ denotes lower alkoxy($C_1$ to $C_6$)carbonyl or cyano and
$R^5$ denotes hydrogen or lower alkyl($C_1$ to $C_6$), in the presence of an inert solvent, if appropriate in the presence of a base, and, in the case of preparation of compounds substituted by hydroxyl in the 4-, 5-, 6- or 7-position, the corresponding benzyloxy compounds are then hydrogenated in the presence of a catalyst in an inert solvent, if appropriate in the presence of an acid, and, in the case of the preparation of N-indolylethylcarbonyl compounds, the corresponding nitriles or alkoxycarbonyls are then hydrolysed, and, in the case of preparation of salts, the products are then reacted with a corresponding base.

5. Process according to Claim 4, characterized in that it is carried out in the temperature range from –20°C to 100°C.

6. Process according to Claims 4 and 5, characterized in that, in the preparation of the hydroxy compounds, the hydrogenation is carried out in the presence of a noble metal catalyst.

7. Process according to Claim 4 and 5, characterized in that, in the preparation of the carboxyl compounds, the hydrolysis is carried out in the presence of an alkali metal or alkaline earth metal hydroxylate or alcoholate.

8. Medicaments containing one or more 4-indolyl-ethyl-sulphonic acid amides according to Claims 1 and 2.

9. Use of N-indolyl-ethyl-sulphonic acid amides according to Claims 1 and 2 for the preparation of medicaments.

10. Use of N-indolyl-ethyl-sulphonic acid amides according to Claims 1 and 2 for the preparation of medicaments for combating and treating thromboses, thromboembolisms, ischaemias, allergies and asthmatic disorders.

## Revendications

1. N-indolyléthyl-sulfonamides de formule:

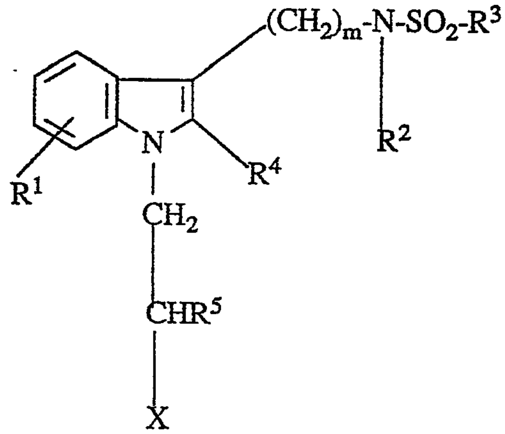

(I),

dans laquelle
$R^1$ représente l'hydrogène, le fluor, le chlore, le brome, un groupe trifluorométhyle, carboxy, (alcoxy inférieur en $C_1$–$C_6$)-carbonyle, le groupe

$S(O)_n-R^6$

dans lequel
$R^6$ représente un groupe alkyle inférieur en $C_1$–$C_6$ ou phényle et
n est égal à 0, 1 ou 2, le groupe

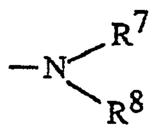

dans lequel
$R^7$ et $R^8$, ayant des significations identiques ou différentes, représentent chacun l'hydrogène, un groupe alkyle inférieur en $C_1$–$C_6$, phényle, benzyle ou acétyle, ou bien le groupe

$-O-R^9$

dans lequel
$R^9$ représente l'hydrogène, un groupe alkyle inférieur en $C_1$–$C_6$, phényle, aralkyle en $C_7$–$C_{10}$, $-SO_2$-alkyle inférieur en $C_1$–$C_6$, $-SO_2$-phényle, $-SO_2$-benzyle ou trifluorométhyle, ou bien un groupe alkyle inférieur en $C_1$–$C_6$, alcényle inférieur en $C_2$–$C_6$, cyclopentyle ou cyclohexyle éventuellement substitué par des groupes carboxy, (alkoxy inférieur en $C_1$–$C_6$)-carbonyle, le fluor, le chlore, le brome et/ou des groupes cyano,
$R^2$ représente l'hydrogène,
$R^3$ représente un groupe alkyle inférieur en $C_1$–$C_6$, alcényle inférieur en $C_2$–$C_6$, cyclopentyle, cyclohexyle, un groupe phényle ou naphtyle portant éventuellement jusqu'à quatre substituants choisis parmi le fluor, le chlore, le brome, les groupes cyano, hydroxy, trifluorométhyle, trifluorométhoxy, alkyle inférieur en $C_1$–$C_6$, alcényle inférieur en $C_2$–$C_6$ eux-mêmes éventuellement substitués par des groupes carboxy ou (alkoxy en $C_1$–$C_4$)-carbonyle, les groupes alcoxy inférieur en $C_1$–$C_6$, carboxyle, (alcoxy en $C_1$–$C_4$)-carboxyle, benzyloxy, benzylthio, phényle, phénoxy et/ou le groupe

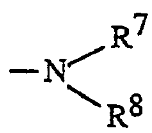

dans lequel
$R^7$ and $R^8$ ont les significations indiquées ci-dessus, ou bien, un groupe pyridyle, thiényle, furyle, pyrimidyle, pipérazinyle, pipéridinyle, morpholinyle, thiomorpholinyle, quinoléyle, isoquinoléyle, imidazolyle, triazolyle, tétrazolyle, thiadiazolyle, pyrrolyle, benzimidazolyle, benzothiadiazolyle, indolyle, indolonyle, thiazolyle, isothiazolyle, benzothiazolyle, benzoisothiazolyle, benzoisothiazolonyle portant éventuellement jusqu'à quatre substituants choisis parmi le fluor, le chlore, le brome, les groupes amino, diméthylamino, acétylamino, trifluorométhyle, trifluorométhoxy, alkyle en $C_1$–$C_4$ et/ou alcoxy en $C_1$–$C_4$,

$R^4$ représente l'hydrogène, un groupe alkyle inférieur en $C_1$–$C_6$, hydroxyalkyle inférieur en $C_1$–$C_6$, alcényle inférieur en $C_2$–$C_6$, (alkyle inférieur en $C_1$–$C_6$)-carbonyle, cyano, un groupe phényle, pyridyle, thiényle, furyle, pyrimidyle, imidazolyle ou pyrrolyle portant éventuellement jusqu'à trois substituants choisis parmi le fluor, le chlore, les groupes méthyle, méthoxy et/ou trifluorométhyle,
$R^5$ représente l'hydrogène ou un groupe alkyle inférieur en $C_1$–$C_6$,
X représente un groupe carboxy et
m est égal à 2,
et leurs sels.

2. N-indolyléthyl-sulfonamides selon la revendication 1, dans lesquels $R^1$ représente l'hydrogène, le fluor, le chlore, le brome, un groupe trifluorométhyle, le groupe

$$-S(O)_n-R^6$$

dans lequel
$R^6$ représente un groupe méthyle, éthyle ou phényle et
n est égal à 0 ou 2,
un groupe amino, méthylamino, diméthylamino, acéthylamino, le groupe

$$-O-R^9$$

dans lequel
$R^9$ représente l'hydrogène, un groupe alkyle en $C_1$–$C_4$, phényle ou benzyle, ou bien un groupe alkyle en $C_1$–$C_4$ éventuellement substitué par le fluor, le chlore et/ou des groupes cyano,
$R^2$ représente l'hydrogène,
$R^3$ représente un groupe alkyle en $C_1$–$C_4$, un groupe phényle ou naphtyle portant éventuellement jusqu'à trois substituants choisis parmi le fluor, le chlore, le brome, les groupes cyano, hydroxy, trifluorométhyle, carboxyvinyle, alkyle en $C_1$–$C_4$, alcoxy en $C_1$–$C_4$, méthoxy- ou éthoxy-carbonylalkyle en $C_1$–$C_4$, phényle, phénoxy, amino, diméthylamino et/ou acétylamino, ou un groupe pyridyle, thiényle, furyle, pyrimidyle, pipérazinyle, pipéridinyle, morpholinyle ou quinoléyle, benzoisothiazolonyle, benzothiazolyle ou indolonyle éventuellement substitué par des groupes alkyle en $C_1$–$C_4$, le fluor, le chlore, des groupes amino, diméthylamino,
$R^4$ représente l'hydrogène, un groupe méthyle, éthyle, hydroxyméthyle, hydroxyéthyle, méthylcarbonyle, cyano ou phényle,
$R^5$ représente l'hydrogène ou un groupe alkyle en $C_1$–$C_4$,
X représente un groupe carboxy et
m est égal à 2,
et leurs sels.

3. Produits intermédiaires de formule:

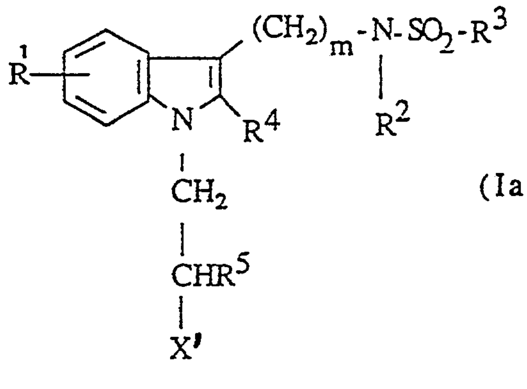

(Ia)

dans laquelle
$R^1$ représente l'hydrogène, le fluor, le chlore, le brome, un groupe trifluorométhyle, carboxy, (alcoxy inférieur en $C_1$–$C_6$)-carbonyle, le groupe

$$-S(O)_n-R^6$$

dans lequel
$R^6$ représente un groupe alkyle inférieur en $C_1$–$C_6$ ou un groupe phényle et
n est égal à 0, 1 ou 2, le groupe

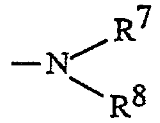

dans lequel
$R^7$ and $R^8$, ayant les significations identiques ou différentes, représentent chacun l'hydrogène, un groupe alkyle inférieur en $C_1$–$C_6$, phényle, benzyle ou acétyle, ou bien le groupe

$$-O-R^9$$

dans lequel
$R^9$ représente l'hydrogène, un groupe alkyle inférieur en $C_1$–$C_6$, phényle, aralkyle en $C_7$–$C_{10}$, $-SO_2$-alkyle inférieur en $C_1$–$C_6$, $-SO_2$-phényle, $-SO_2$-benzyle ou trifluorométhyle, ou bien un groupe alkyle inférieur en $C_1$–$C_6$, alcényle inférieur en $C_2$–$C_6$, cyclopentyle ou cyclohexyle éventuellement substitué par des groupes carboxy, (alcoxy inférieur en $C_1$–$C_6$)-carbonyle, le fluor, le chlore, le brome et/ou des groupes cyano,
$R^2$ représente un groupe alkyle inférieur en $C_1$–$C_6$, alcényle inférieur en $C_1$–$C_6$, cyclopentyle ou cyclohexyle éventuellement substitué par le fluor, le chlore, le brome, des groupes carboxy, (alcoxy inférieur en $C_1$–$C_6$)-carbonyle et/ou cyano,
$R^3$ représente un groupe alkyle inférieur en $C_1$–$C_6$, alcényle inférieur en $C_2$–$C_6$, cyclopentyle, cyclohexyle, un groupe phényle ou naphtyle portant éventuellement jusqu'à quatre substituants choisis parmi le fluor, le chlore, le brome, les groupes cyano, hydroxy, trifluorométhyle, trifluorométhoxy, alkyle inférieur en $C_1$–$C_6$, alcényle inférieur en $C_1$–$C_6$ eux-mêmes éventuellement substitués par des groupes carboxy ou (alcoxy en $C_1$–$C_4$)-carbonyle, les groupes alcoxy inférieur en $C_1$–$C_6$, carboxyle, (alcoxy en $C_1$–$C_4$)-carboxyle, benzyloxy, benzylthio, phényle, phénoxy et/ou le groupe

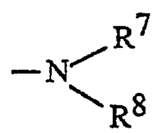

dans lequel
$R^7$ et $R^8$ ont les significations indiquées ci-dessus, ou bien, un groupe pyridyle, thiényle, furyle, pyrimidyle, pipérazinyle, pipéridinyle, morpholinyle, thiomorpholinyle, quinoléyle, isoquinoléyle, imidazolyle, triazolyle, tétrazolyle, thiadiazolyle, pyrrolyle, benzimidazolyle, benzothiadiazolyle, indolyle, indolonyle, thiazolyle, isothiazolyle, benzothiazolyle, benzoisothiazolyle, benzoisothiazolonyle portant éventuellement jusqu'à quatre substituants choisis parmi le fluor, le chlore, le brome, les groupes amino, diméthylamino, acétylamino, trifluorométhyle, trifluorométhoxy, alkyle en $C_1$–$C_4$ et/ou alcoxy en $C_1$–$C_4$,
$R^4$ représente l'hydrogène, un groupe alkyle inférieur en $C_1$–$C_6$, hydroxyalkyle inférieur en $C_1$–$C_6$, alcényle inférieur en $C_2$–$C_6$, (alkyle inférieur en $C_1$–$C_6$)-carbonyle, cyano, un groupe phényle, pyridyle, thiényle, furyle, pyrimidyle, imidazolyle ou pyrrolyle portant éventuellement jusqu'à trois substituants choisis parmi le fluor, le chlore, les groupes méthyle, méthoxy et/ou trifluorométhyle,
$R^5$ représente l'hydrogène ou un groupe alkyle inférieur en $C_1$–$C_6$,
X' représente un groupe (alcoxy inférieur en $C_1$–$C_6$)-carbonyle ou cyano,
m est égal à 2,
et leurs sels.

4. Procédé de préparation des N-indolyléthyl-sulfonamides et de leurs sels selon la revendication 1, caractérisé en ce que l'on fait réagir des indolylalkylamines de formule

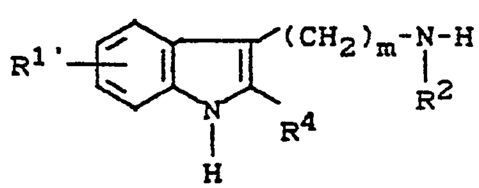

(II),

dans lequelle
$R^{1'}$ représente l'hydrogène, le fluor, le chlore, le brome, un groupe trifluorométhyle, carboxy, (alcoxy inférieur en $C_1$–$C_6$)-carbonyle, le groupe

$$-S(O)_n-R^6$$

dans lequel
$R^6$ représente un groupe alkyle inférieur en $C_1$–$C_6$ ou phényle et
n est égal à 0, 1 ou 2, le groupe

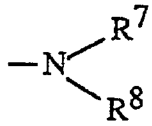

dans lequel

$R^7$ et $R^8$, ayant des significations identiques ou différentes, représentent chacun l'hydrogène, un groupe alkyle inférieur en $C_1$–$C_6$, phényle, benzyle ou acétyle, ou bien le groupe

$$-O-R^9$$

dans lequel

$R^9$ représente un groupe alkyle inférieur en $C_1$–$C_6$, phényle, aralkyle en $C_7$–$C_{10}$, $-SO_2$-alkyle inférieur en $C_1$–$C_6$, $-SO_2$-phényle, $-SO_2$-benzyle ou trifluorométhyle, ou un groupe alkyle inférieur en $C_1$–$C_6$, alcényle inférieur en $C_1$–$C_6$, cyclopentyle ou cyclohexyle éventuellement substitué par des groupes carboxy, (alcoxy inférieur en $C_1$–$C_6$)-carbonyle, le fluor, le chlore, le brome et/ou des groupes cyano,

$R^2$ représente l'hydrogène,

$R^4$ représente l'hydrogène, un groupe alkyle inférieur en $C_1$–$C_6$, hydroxyalkyle inférieur en $C_1$–$C_6$, alcényle inférieur en $C_2$–$C_6$, (alkyle inférieur en $C_1$–$C_6$)-carbonyle, cyano, un groupe phényle, pyridyle, thiényle, furyle, pyrimidyle, imidazolyle ou pyrrolyle portant éventuellement jusqu'à trois substituants choisis parmi le fluor, le chlore, les groupes méthyle, méthoxy et/ou trifluorométhyle, et

m est égal à 2,

avec des halogénures d'acides sulfoniques de formule

$$R^3-SO_2-Y \qquad (III),$$

dans laquelle

$R^3$ représente un groupe alkyle inférieur en $C_1$–$C_6$, alcényle inférieur en $C_2$–$C_6$, cyclopentyle, cyclohexyle, un groupe phényle ou naphtyle portant éventuellement jusqu'à quatre substituants choisis parmi le fluor, le chlore, le brome, les groupes cyano, hydroxy, trifluorométhyle, trifluorométhoxy, alkyle inférieur en $C_1$–$C_6$, alcényle inférieur en $C_2$–$C_6$ eux-mêmes éventuellement substitués par des groupes carboxy ou (alcoxy en $C_1$–$C_4$)-carbonyle, les groupes alcoxy inférieur en $C_1$–$C_6$, carboxyle, (alcoxy en $C_1$–$C_4$)-carboxyle, benzyloxy, benzylthio, phényle, phénoxy et/ou le groupe

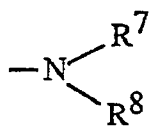

dans lequel

$R^7$ et $R^8$ ont les significations indiquées ci-dessus, ou bien, un groupe pyridyle, thiényle, furyle, pyrimidyle, pipérazinyle, pipéridinyle, morpholinyle, thiomorpholinyle, quinoléyle, isoquinoléyle, imidazolyle, triazolyle, tétrazolyle, thiadiazolyle, pyrrolyle, benzimidazolyle, benzothiadiazolyle, indolyle, indolonyle, thiazolyle, isothiazolyle, benzothiazolyle, benzoisothiazolyle, benzoisothiazolonyle portant éventuellement jusqu'à quatre substituants choisis parmi le fluor, le chlore, le brome, les groupes amino, diméthylamino, acétylamino, trifluorométhyle, trifluorométhoxy, alkyle en $C_1$–$C_4$ et/ou alcoxy en $C_1$–$C_4$, et

Y représente un halogène,

en présence d'un solvant inerte, éventuellement en présence d'une base, après quoi on alkyle à l'aide d'un composé oléfinique de formule

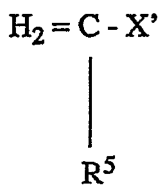

(IV),

dans laquelle

X' représente un groupe (alcoxy inférieur en $C_1$–$C_6$)-carbonyle ou cyano et

$R^5$ représente l'hydrogène ou un groupe alkyle inférieur en $C_1$–$C_6$, en présence d'un solvant inerte, éventuellement en présence d'une base, ce qui donne les produits intermédiaires de formule Ia) puis, dans le cas où on prépare des composés substitués en position 4, 5, 6 ou 7 par un groupe hydroxy, on hydrogène les dérivés benzyloxylés correspondants en présence d'un catalyseur dans un solvant inerte, éventuellement en présence d'un acide, et, dans le cas où on prépare des dérivés N-indolyléthylcarbonylés, on saponifie les nitriles ou les dérivés alcoxycarbonylés correspondants,

et dans le cas où on prépare des sels, on fait réagir avec la base correspondante.

5. Procédé selon la revendication 4, caractérisé en ce que l'on opère dans un intervalle de température allant de –20 à 100°C.

6. Procédé selon les revendications 4 et 5, caractérisé en ce que, pour la préparation des composés hydroxylés, on hydrogène en présence d'un catalyseur à base de métal noble.

7. Procédé selon les revendications 4 et 5, caractérisé en ce que, à la préparation des dérivés carboxylés, on saponifie en présence d'un hydroxylate ou d'un alcoolate de métal alcalin ou alcalino-terreux.

8. Médicament contenant un ou plusieurs 4-indolyléthyl-sulfonamides selon les revendications 1 et 2.

9. Utilisation des N-indolyléthyl-sulfonamides selon les revendications 1 et 2 pour la préparation de médicaments.

10. Utilisation des N-indolyléthyl-sulfonamides selon les revendications 1 et 2 pour la préparation de médicaments pour le traitement ou la prévention des thromboses, des thrombo-embolies, des ischémies, des allergies et des troubles asthmatiques.